(19) Europäisches Patentamt
European Patent Office
Office européen des brevets

(11) EP 1 674 101 B1

(12) FASCICULE DE BREVET EUROPEEN

(45) Date de publication et mention de la délivrance du brevet:
**14.11.2007 Bulletin 2007/46**

(51) Int Cl.:
***A61K 31/4741** (2006.01)* ***A61P 35/00** (2006.01)*

(21) Numéro de dépôt: **05292744.9**

(22) Date de dépôt: **21.12.2005**

(54) **Dérivés cinnamates de benzo [b]pyrano[3,2-h]acridin-7-one, leur procédé de préparation et les compositions pharmaceutiques qui les contiennent**

Benzo[b]pyrano[3,2-h]acridin-7-one-zimtsäurederivate, Verfahren zu deren Herstellung und pharmazeutische Zusammensetzungen diese enthaltend

Cinnamates of Benzo[b]pyrano[3,2-h]acridin-7-one, process for preparation thereof and their pharmaceutical composition

(84) Etats contractants désignés:
**AT BE BG CH CY CZ DE DK EE ES FI FR GB GR HU IE IS IT LI LT LU LV MC NL PL PT RO SE SI SK TR**
Etats d'extension désignés:
**AL BA HR MK YU**

(30) Priorité: **22.12.2004 FR 0413682**

(43) Date de publication de la demande:
**28.06.2006 Bulletin 2006/26**

(73) Titulaires:
- **Les Laboratoires Servier**
  **92415 Courbevoie Cedex (FR)**
- **CENTRE NATIONAL DE LA RECHERCHE SCIENTIFIQUE (CNRS)**
  **75794 Paris Cedex 16 (FR)**
- **UNIVERSITE RENE DESCARTES (PARIS V)**
  **F-75270 Paris Cédex 06 (FR)**

(72) Inventeurs:
- **Koch, Michel**
  **78170 La Celle Saint Cloud (FR)**
- **Tillequin, François**
  **75014 Paris (FR)**
- **Michel, Sylvie**
  **75002 Paris (FR)**
- **Hickman, John**
  **75017 Paris (FR)**
- **Pierre, Alain**
  **78580 Les Alluets le Roi (FR)**
- **Leonce, Stéphane**
  **78000 Versailles (FR)**
- **Pfeiffer, Bruno**
  **95320 Saint Leu La Forêt (FR)**
- **Kraus-Berthier, Laurence**
  **92700 Colombes (FR)**

(56) Documents cités:
**EP-A- 1 061 081**
**EP-A- 1 297 835**
**WO-A-99/32491**

Remarques:
Le dossier contient des informations techniques présentées postérieurement au dépôt de la demande et ne figurant pas dans le présent fascicule.

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

EP 1 674 101 B1

Printed by Jouve, 75001 PARIS (FR)

## Description

La présente invention concerne de nouveaux dérivés cinnamates de benzo[*b*]pyrano[3,2-*h*] acridin-7-one, leur procédé de préparation et les compositions pharmaceutiques qui les contiennent.

Les composés de l'invention constituent des dérivés de l'acronycine qui est un alcaloïde présentant des propriétés antitumorales mises en évidence dans des modèles expérimentaux (J. Pharm.. Sci., 1966, 55 (8), 758-768). Cependant, malgré un spectre d'activité assez large, l'acronycine est peu puissante et modérément active. De plus, ce produit présente une faible solubilité limitant sa biodisponibilité ainsi que son utilisation dans des compositions pharmaceutiques administrables par voie intraveineuse.

Diverses modifications ont été réalisées sur cette molécule, comme celles décrites dans J. Med. Chem., 1996, 39, 4762-4766, EP 1 042 326, EP 1 061 081 ou EP 1 297 835, et qui ont permis d'améliorer significativement la puissance, l'efficacité antitumorale et la solubilité de ces produits. Néanmoins, les besoins de la thérapeutique anticancéreuse exigent le développement constant de nouveaux agents antitumoraux, dans le but d'obtenir des médicaments à la fois plus actifs et mieux tolérés. Plus particulièrement, les tumeurs solides posent un problème majeur à la chimiothérapie anticancéreuse, de par leur résistance intrinsèque et/ou acquise, aux produits existants. Il est donc primordial d'avoir accès à une gamme la plus large possible de produits exprimant une activité cytotoxique forte afin de pouvoir disposer de traitements des plus efficaces sur l'ensemble des affections tumorales.

Les composés de l'invention, outre le fait qu'ils soient nouveaux, présentent une activité cytotoxique *in vitro* et *in vivo* surprenante et supérieure à celle observée jusqu'ici. Ainsi, les composés découverts par la Demanderesse possèdent des propriétés antitumorales qui les rendent particulièrement utiles pour le traitement des cancers. Parmi les types de cancers qui peuvent être traités par les composés de la présente invention, on peut citer à titre non limitatif les adénocarcinomes et carcinomes, sarcomes, gliomes et leucémies.

Plus particulièrement, la présente invention concerne les composés de formule (I) :

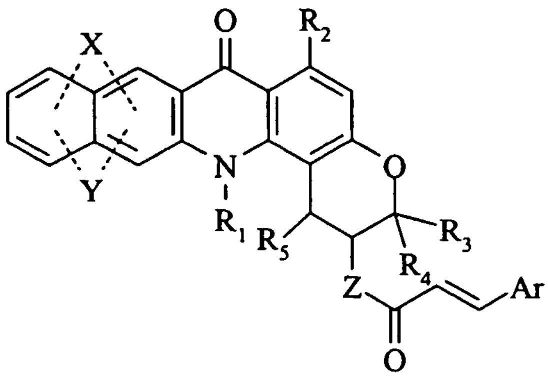

(I)

dans laquelle :

- X et Y, identiques ou différents, indépendamment l'un de l'autre, représentent chacun un groupement choisi parmi :

  - atome d'hydrogène, halogène,
  - groupement hydroxy, alkoxy ($C_1$-$C_6$) linéaire ou ramifié, nitro, cyano, alkyle ($C_1$-$C_6$) linéaire ou ramifié, éventuellement substitué par un ou plusieurs groupements choisis parmi hydroxy et halogène, et alkényle ($C_2$-$C_6$) linéaire ou ramifié, ou
  - groupement de formule $-NR_aR_b$ dans lequel :

    $R_a$ et $R_b$, identiques ou différents, indépendamment l'un de l'autre, représentent chacun un groupement choisi parmi atome d'hydrogène, ou groupement alkyle (Ci-$C_6$) linéaire ou ramifié,
    ou $R_a$ et $R_b$ forment ensemble avec l'atome d'azote qui les portent, un hétérocycle de 5 à 7 chaînons monocyclique, contenant éventuellement au sein du système cyclique un second hétéroatome choisi parmi oxygène et azote,

  étant entendu que les substituants X et Y peuvent être présents, indépendamment l'un de l'autre, sur l'un ou l'autre des deux cycles benzéniques adjacents,

- Z représente un atome d'oxygène ou $NR_c$ dans lequel $R_c$, représente un groupement choisi parmi atome d'hydrogène,

groupement alkyle ($C_1$-$C_6$) linéaire ou ramifié, ou un groupement aryle, arylalkyle ($C_1$-$C_6$) linéaire ou ramifié,

- Ar représente un groupement aryle ou hétéroaryle,
- $R_1$ représente un atome d'hydrogène ou un groupement alkyle ($C_1$-$C_6$) linéaire ou ramifié,
- $R_2$ représente un groupement choisi parmi atome d'hydrogène, groupement alkyle ($C_1$-$C_6$) linéaire ou ramifié, -$OR_a$ ; $NR_aR_b$ dans lesquels $R_a$ et $R_b$ sont tels que définis précédemment,
- $R_3$, $R_4$, identiques ou différents, indépendamment l'un de l'autre, représentent un atome d'hydrogène ou un groupement alkyle ($C_1$-$C_6$) linéaire ou ramifié,
- $R_5$ représente un groupement choisi parmi :

  1) atome d'hydrogène,

  2) groupement $OR_c$, $NR_cR_d$ dans lesquels :

  - $R_c$, est tel que défini précédemment et $R_d$, a les mêmes définitions que $R_c$,

  3) $W_1$-C($W_2$)-U-V dans lequel :

    α) $W_1$ représente un atome d'oxygène ou $NR_c$ (dans lequel $R_c$ est tel que défini précédemment),
    β) $W_2$ représente un atome d'oxygène,
    γ) U représente une liaison simple ou une chaîne alkylène ($C_1$-$C_8$) linéaire ou ramifiée ou une chaîne alkénylène ($C_2$-$C_8$) linéaire ou ramifiée,
    δ) V représente un groupement choisi parmi :

    - atome d'hydrogène,
    - groupement aryle, hétéroaryle,
    - groupements $OR_c$, $CO_2R_c$, $COR_c$, $CONR'_aR'_b$, $NR'_aR'_b$, $N(R_c)$-$CO_2R'_c$, $N(R_c)$-$COR'_c$, dans lesquels $R_c$ est tel que défini précédemment, $R'_c$ a les mêmes définitions que $R_c$ et, $R'_a$ et $R'_b$, identiques ou différents, indépendamment l'un de l'autre, représentent chacun un groupement choisi parmi atome d'hydrogène, groupement alkyle ($C_1$-$C_6$) linéaire ou ramifié, aryle, arylalkyle ($C_1$-$C_6$) linéaire ou ramifié, ou $R'_a$ et $R'_b$ forment ensemble avec l'atome d'azote qui les portent, un hétérocycle de 5 à 7 chaînons, monocyclique, contenant éventuellement au sein du système cyclique un second hétéroatome choisi parmi oxygène et azote,

  4) $W_1$-C($W_2$)-$W_3$-$T_1$ dans lequel :

    α) $W_1$ et $W_2$ sont tels que définis précédemment,
    β) $W_3$ représente un atome d'oxygène ou $NR_c$ dans lequel $R_c$ est tel que défini précédemment,
    γ) $T_1$ représente un groupement choisi parmi :

    - atome d'hydrogène,
    - alkyle ($C_1$-$C_6$) linéaire ou ramifié,
    - alkényle ($C_2$-$C_6$) linéaire ou ramifié,
    - aryle, arylalkyle ($C_1$-$C_6$) linéaire ou ramifié,
    - chaîne alkylène ($C_1$-$C_6$) linéaire ou ramifiée ou une chaîne alkénylène ($C_2$-$C_6$) linéaire ou ramifiée, chacune étant substituée par un groupement $OR_c$ dans lequel $R_c$ est tel que défini précédemment ou par $NR'_aR'_b$ dans lequel $R'_a$ et $R'_b$ sont tels que définis précédemment,

  5) Z-CO-CH=CHAr dans lequel Z et Ar sont tels que définis précédemment,

leurs énantiomères, diastéréoisomères, lorsqu'ils existent, ainsi que leurs sels d'addition à un acide ou à une base pharmaceutiquement acceptable, ainsi que leurs hydrates et leurs solvats,

étant entendu que :

par aryle, on comprend un groupement phényle ou naphtyle, comportant éventuellement un ou plusieurs substituants, identiques ou différents, choisis parmi alkyle ($C_1$-$C_6$) linéaire ou ramifié éventuellement substitué par un ou plusieurs groupements hydroxy ou halogène, hydroxy, halogène, carboxy, nitro, amino, monoalkylamino($C_1$-$C_6$)linéaire ou ramifié, ou dialkylamino($C_1$-$C_6$)linéaire ou ramifié, alkoxy ($C_1$-$C_6$) linéaire ou ramifié, acyle ($C_1$-$C_6$) linéaire ou ramifié, et alkylcarbonyloxy ($C_1$-$C_6$) linéaire ou ramifié,

par hétéroaryle, on entend un groupement de 5 à 12 chaînons, soit monocyclique aromatique, soit bicyclique dont l'un au moins des cycles possède un caractère aromatique, et contenant un, deux ou trois hétéroatomes choisis parmi oxygène, azote ou soufre, étant entendu que l'hétéroaryle peut être éventuellement substitué par un ou plusieurs atomes ou groupements, identiques ou différents, choisis parmi les atomes d'halogène et les groupements alkyle ($C_1$-$C_6$) linéaire ou ramifié, éventuellement substitué par un ou plusieurs groupements hydroxy ou halogène, ou hydroxy, alkoxy ($C_1$-$C_6$) linéaire ou ramifié, ou amino (substitué éventuellement par un ou deux groupements alkyle ($C_1$-$C_6$) linéaire ou ramifié).

Parmi les groupements hétéroaryle, on peut citer à titre non limitatif les groupements thiényle, pyridyle, furyle, pyrrolyle, imidazolyle, oxazolyle, isoxazolyle, thiazolyle, isothiazolyle, quinolyle, isoquinolyle, pyrimidinyle.

Parmi les hétérocycles de 5 à 7 chaînons, monocyclique, contenant éventuellement au sein du système cyclique un second hétéroatome choisi parmi oxygène et azote, on peut citer à titre non limitatif les groupements pyrrolidinyle, isoxazolidinyle, oxazolidinyle, pyrazolidinyle, imidazolidinyle, pipéridinyle, oxazinanyle, morpholinyle, hexahydropyridazinyle, hexahydropyrimidinyle, pipérazinyle, azépanyle, oxazépanyle, diazépanyle.

Parmi les acides pharmaceutiquement acceptables, on peut citer à titre non limitatif les acides chlorhydrique, bromhydrique, sulfurique, phosphonique, acétique, trifluoroacétique, lactique, pyruvique, malonique, succinique, glutarique, fumarique, tartrique, maléique, citrique, ascorbique, oxalique, méthane sulfonique, benzène sulphonique, camphorique, la lysine, etc...

Parmi les bases pharmaceutiquement acceptables, on peut citer à titre non limitatif l'hydroxyde sodium, l'hydroxyde de potassium, la triéthylamine, la tertbutylamine, etc...

Les substituants X et Y préférés selon l'invention sont l'atome d'hydrogène.

Les substituants $R_1$, $R_3$ et $R_4$ préférés selon l'invention sont le groupement alkyle ($C_1$-$C_6$) linéaire ou ramifié.

Le substituant $R_2$ préféré selon l'invention est le groupement -$OR_a$ dans lequel $R_a$ est tel que défini dans la formule (I).

Le substituant $R_5$ préféré selon l'invention est le groupement -$OR_c$ dans lequel $R_c$ est tel que défini dans la formule (I) et le groupement $W_1$-C($W_2$)-U-V dans lequel $W_1$, $W_2$, U et V sont tels que définis dans la formule (I).

Encore plus préférentiellement, le substituant $R_5$ préféré selon l'invention est le groupement -$OR_c$ dans lequel $R_c$ représente un atome d'hydrogène et le groupement $W_1$-C($W_2$)-U-V dans lequel $W_1$ et $W_2$ représentent chacun un atome d'oxygène, U représente une chaîne alkylène ($C_1$-$C_8$) linéaire ou ramifiée et V représente un atome d'hydrogène.

Le substituant Z préféré selon l'invention est l'atome d'oxygène.

Le substituant Ar préféré selon l'invention est un groupement phényle éventuellement substitué.

D'une façon particulièrement avantageuse, les composés préférés de l'invention sont le :

- ➢ (±)-*cis*-2-Cinnamoyloxy-1-hydroxy-6-méthoxy-3,3,14-triméthyl-1,2,3,14,-tétrahydro-1*H*-benzo[*b*]pyrano[3,2-*h*]acridin-7-one,
- ➢ (±)-*cis*-1-Acétoxy-2-cinnamoyloxy-6-méthoxy-3,3,14-triméthyl-1,2,3,14,-tétrahydro-1*H*-benzo[*b*]pyrano[3,2-*h*]acridin-7-one,
- ➢ (±)-*cis*-2-(4-Chlorocinnamoyloxy)-1-hydroxy-6-méthoxy-3,3,14-triméthyl-1,2,3,14,-tétrahydro-1*H*-benzo[*b*]pyrano[3,2-*h*]acridin-7-one,
- ➢ (±)-*cis*-1-Acétoxy-2-(4-chlorocinnamoyloxy)-6-méthoxy-3,3,14-triméthyl-1,2,3,14,-tétrahydro-1*H*-benzo[*b*]pyrano[3,2-*h*]acridin-7-one,
- ➢ (±)-*cis*-2-(2-Chlorocinnamoyloxy)-1-hydroxy-6-méthoxy-3,3,14-triméthyl-1,2,3,14,-tétrahydro-1*H*-benzo[*b*]pyrano[3,2-*h*]acridin-7-one,
- ➢ (±)-*cis*-1-Acétoxy-2-(2-chlorocinnamoyloxy)-6-méthoxy-3,3,14-triméthyl-1,2,3,14,-tétrahydro-1*H*-benzo[*b*]pyrano[3,2-*h*]acridin-7-one,
- ➢ (±)-*cis*-2-(3-Chlorocinnamoyloxy)-1-hydroxy-6-méthoxy-3,3,14-triméthyl-1,2,3,14,-tétrahydro-1*H*-benzo[*b*]pyrano[3,2-*h*]acridin-7-one,
- ➢ (±)-*cis*-2-(2,4-Dichlorocinnamoyloxy)-1-hydroxy-6-méthoxy-3,3,14-triméthyl-1,2,3,14,-tétrahydro-1*H*-benzo[*b*]pyrano[3,2-*h*]acridin-7-one,
- ➢ (±)-*cis*-1-Acétoxy-2-(2,4-dichlorocinnamoyloxy)-6-méthoxy-3,3,14-triméthyl-1,2,3,14,-tétrahydro-1*H*-benzo[*b*]pyrano[3,2-*h*]acridin-7-one,
- ➢ (±)-*cis*-2-(3,4-Dichlorocinnamoyloxy)-1-hydroxy-6-méthoxy-3,3,14-triméthyl-1,2,3,14,-tétrahydro-1*H*-benzo[*b*]pyrano[3,2-*h*]acridin-7-one,

➢ (±)-*cis*-1-Acétoxy-2-(3,4-dichlorocinnamoyloxy)-6-méthoxy-3,3,14-triméthyl-1,2,3,14,-tétrahydro-1*H*-benzo[*b*]pyrano[3,2-*h*]acridin-7-one,
➢ (±)-*cis*-2-(4-Bromocinnamoyloxy)-1-hydroxy-6-méthoxy-3,3,14-triméthyl-1,2,3,14,-tétrahydro-1*H*-benzo[*b*]pyrano[3,2-*h*]acridin-7-one,
➢ (±)-*cis*-1-Acétoxy-2-(4-bromocinnamoyloxy)-6-méthoxy-3,3,14-triméthyl-1,2,3,14,-tétrahydro-1*H*-benzo[*b*]pyrano[3,2-*h*]acridin-7-one,
➢ (±)-*cis*-1-Hydroxy-6-méthoxy-2-(4-méthoxycinnamoyloxy)-3,3,14-triméthyl-1,2,3,14,-tétrahydro-1*H*-benzo[*b*]pyrano[3,2-*h*]acridin-7-one,
➢ (±)-*cis*-1-Hydroxy-6-méthoxy-2-(4-nitrocinnamoyloxy)-3,3,14-triméthyl-1,2,3,14,-tétrahydro-1*H*-benzo[*b*]pyrano[3,2-*h*]acridin-7-one,
➢ (±)-*cis*-1-Acétoxy-6-méthoxy-2-(4-nitrocinnamoyloxy)-3,3,14-triméthyl-1,2,3,14,-tétrahydro-1*H*-benzo[*b*]pyrano[3,2-*h*]acridin-7-one.

La présente invention s'étend également au procédé de préparation des composés de formule (I), caractérisé en ce qu'on utilise comme produit de départ un composé de formule (II) :

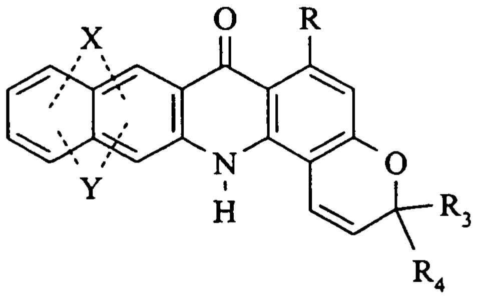

(II)

dans laquelle X, Y, $R_3$ et $R_4$ sont tels que définis précédemment, et R représente un atome d'hydrogène, un groupement hydroxy, un groupement alkyle ($C_1$-$C_6$) linéaire ou ramifié, composé de formule (II) dont l'atome d'azote est substitué ou non, par action d'un halogénure d'alkyle ou d'un sulfate de dialkyle en présence d'un agent de déprotonation, en solvant polaire aprotique ou en condition de transfert de phase, permettant d'obtenir les composés de formule (III) :

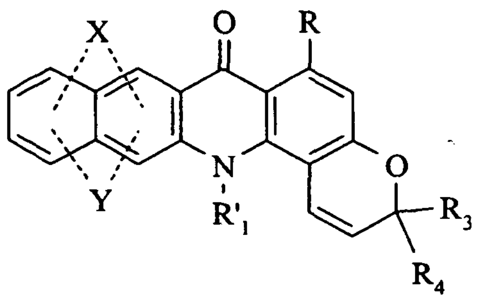

(III)

dans laquelle X, Y, R, $R_3$ et $R_4$ sont tels que définis précédemment, et $R'_1$ représente un groupement alkyle ($C_1$-$C_6$) linéaire ou ramifié,
composés de formule (III) qui sont soumis à l'action d'un agent alkylant selon des conditions classiques de la synthèse organique, pour conduire aux composés de formule (IV) :

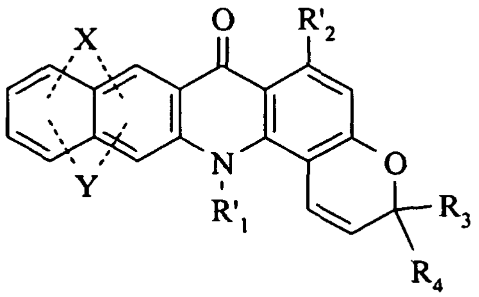

(IV)

dans laquelle X, Y, R'$_1$, $R_3$ et $R_4$ sont tels que définis précédemment et R'$_2$ représente un groupement choisi parmi OR'$_a$ dans lequel R'$_a$ représente un groupement alkyle ($C_1$-$C_6$) linéaire ou ramifié,
composés de formule (IV) qui sont traités, dans le cas où R'$_2$ représente un groupement alkoxy par un composé de formule (V) :

$HNR_aR_b$ (V)

dans laquelle $R_a$ et $R_b$ sont tels que définis dans la formule (I),
pour conduire aux composés de formule (VI) :

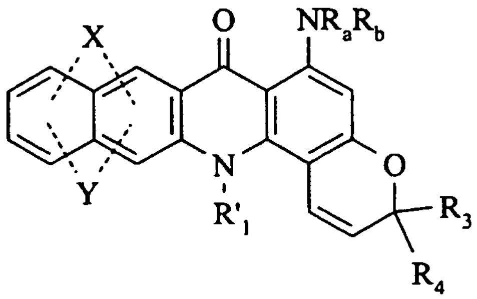

(VI)

dans laquelle X, Y, R'$_1$, $R_3$, $R_4$, $R_a$ et $R_b$ sont tels que définis précédemment,
l'ensemble des composés de formule (II), (III), (IV) et (VI) formant les composés de formule (VII) :

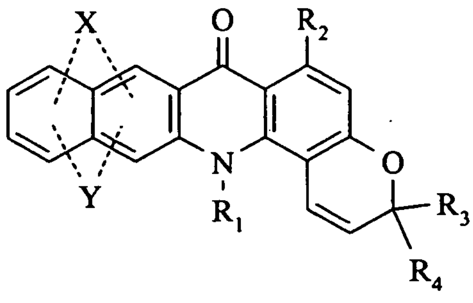

(VII)

dans laquelle X, Y, $R_1$, $R_2$, $R_3$ et $R_4$ sont tels que définis dans la formule (I),
composés de formule (VII) qui sont soumis :

a) soit à l'action du tétroxyde d'osmium en milieu polaire et en présence de 4-méthylmorpholine-N-oxyde, pour conduire aux composés de formule (VIII/a) :

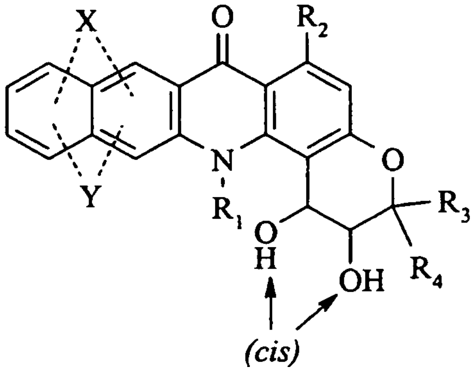

(VIII/a)

dans laquelle X, Y, $R_1$, $R_2$, $R_3$ et $R_4$ sont tels que définis précédemment,

b) soit à l'action de permanganate de potassium en milieu polaire puis à des conditions réductrices en présence de $NaBH_4$, pour conduire au composé de formule (VIII/b) :

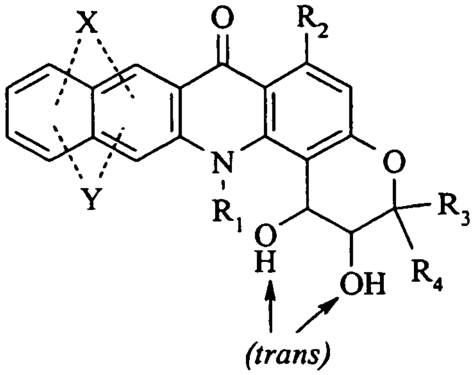

(VIII/b)

dans laquelle X, Y, $R_1$, $R_2$, $R_3$ et $R_4$ sont tels que définis précédemment,
l'ensemble des composés de formules (VIII/a) et (VIII/b) formant les composés de formule (VIII) dont les 2 groupements alcools peuvent être de configuration *cis* ou *trans* l'un par rapport à l'autre :

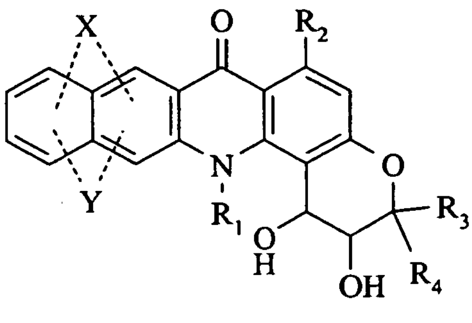

(VIII)

dans laquelle X, Y, $R_1$, $R_2$, $R_3$ et $R_4$ sont tels que définis précédemment,
composés de formule (VIII) qui sont soumis à l'action d'un ou de 2 équivalents d'un anhydride de formule (IX) ou d'un chlorure d'acide de formule (X) :

$[Ar\text{-}CH{=}CH\text{-}C(O)]_2O$ (IX) Ar-HC=CH-C(O)-Cl (X)

dans lesquelles Ar est tel que défini dans la formule (I) pour conduire aux composés de formules ($I/a_1$) ou ($I/a_2$), cas particuliers des composés de formule (I) :

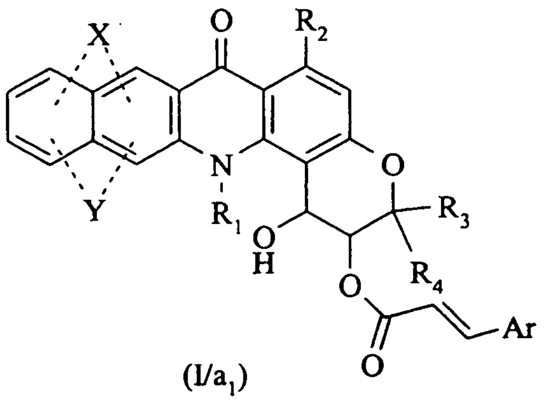

(I/a$_1$)

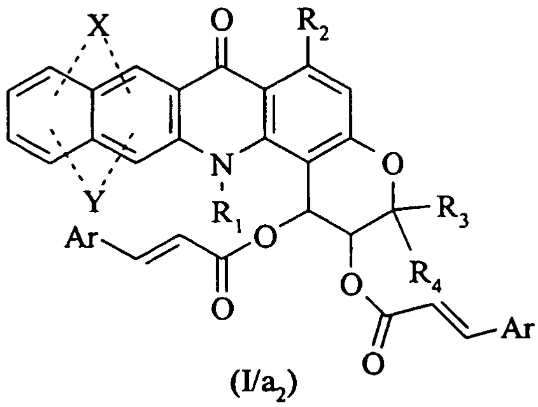

(I/a$_2$)

dans lesquelles X, Y, $R_1$, $R_2$, $R_3$, $R_4$ et Ar sont tels que définis précédemment,

c) soit à l'action de $NaN_3$, en présence d'eau oxygénée, suivi d'une étape de réduction, pour conduire aux composés de formule (XI) :

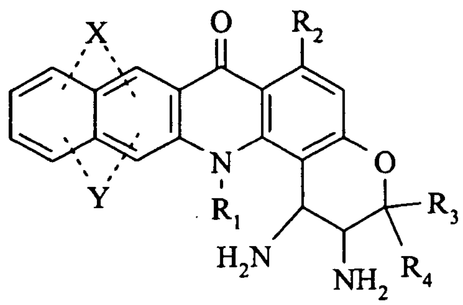

(XI)

dans laquelle X, Y, $R_1$, $R_2$, $R_3$ et $R_4$ sont tels que définis précédemment,
composés de formule (XI) qui sont soumis à l'action des composés de formules (IX) ou (X) tels que définis précédemment dans les mêmes conditions que pour les composés de formule (VIII), pour conduire aux composés de formules (I/b$_1$) ou (I/b$_2$), cas particuliers des composés de formule (I) :

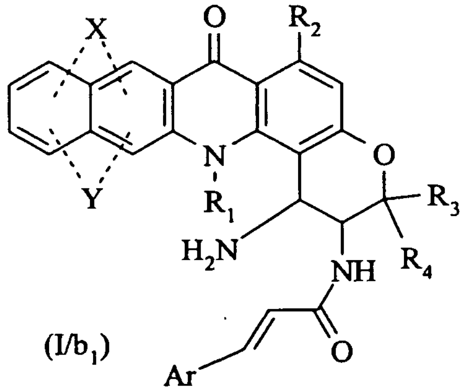

(I/b$_1$)

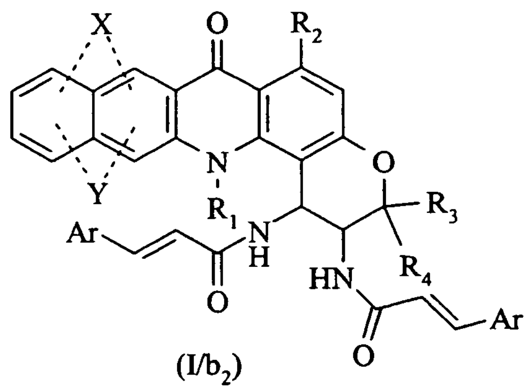

(I/b$_2$)

dans lesquelles X, Y, $R_1$, $R_2$, $R_3$, $R_4$ et Ar sont tels que définis précédemment, composés de formule (I/b$_1$) ou (I/b$_2$) qui sont éventuellement soumis à l'action d'un composé de formule (XII) :

$R'_c$-Hal (XII)

dans laquelle Hal représente un halogène et $R'_c$ représente un groupement choisi parmi alkyle ($C_1$-$C_6$) linéaire ou ramifié,

ou un groupement aryle, arylalkyle ($C_1$-$C_6$) linéaire ou ramifié, pour conduire aux composés de formule (I/$c_1$) ou (I/$c_2$) :

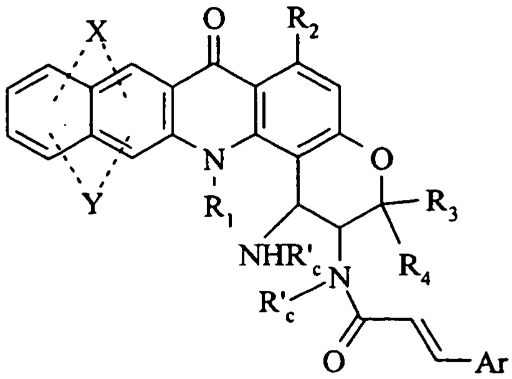

(I/$c_1$)

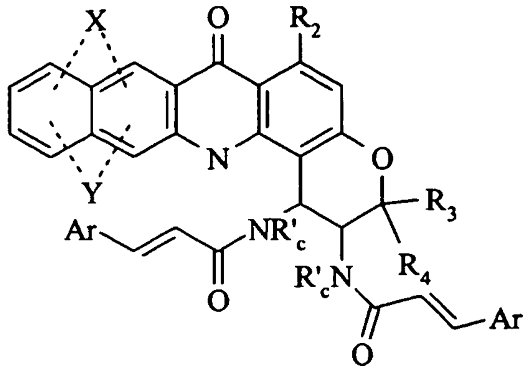

(I/$c_2$)

dans lesquelles X, Y, $R_1$, $R_2$, $R_3$, $R_4$, $R'_c$ et Ar sont tels que définis précédemment,
l'ensemble des composés de formules (I/$a_1$), (I/$b_1$) et (I/$c_1$) et (I/$a_2$), (I/$b_2$) et (I/$c_2$) formant les composés de formules (I/$d_1$) et (I/$d_2$) respectivement,

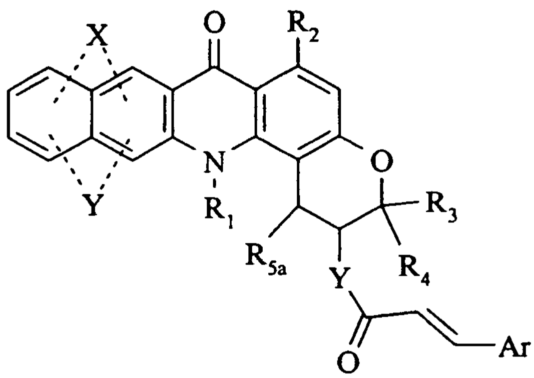

(I/$d_1$)

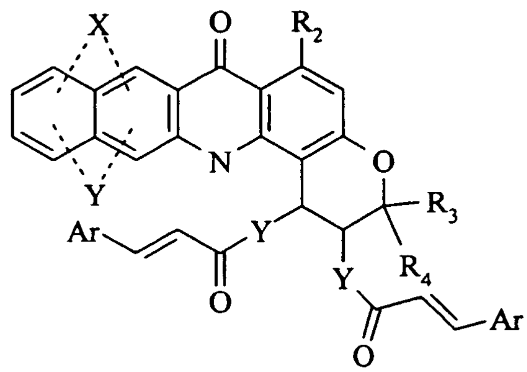

(I/$d_2$)

dans lesquelles X, Y, $R_1$, $R_2$, $R_3$, $R_4$, Y et Ar sont tels que définis dans la formule (I) et $R_{5a}$ représente un groupement hydroxy, $NH_2$ ou $NHR'_c$ dans lequel $R'_c$ est tel que défini précédemment,
les composés de formule (I/$d_1$) qui sont éventuellement soumis :

a) soit à l'action d'un agent alkylant, pour conduire aux composés de formule (I/e), cas particulier des composés de formule (I) :

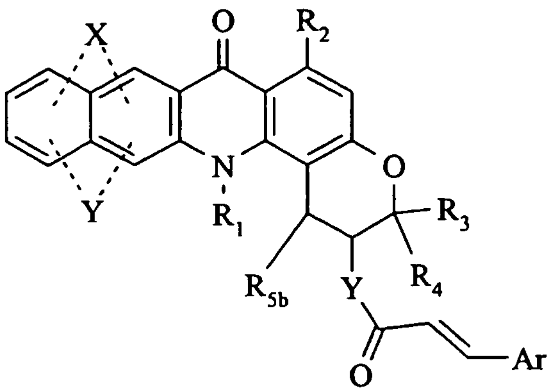

(I/e)

dans laquelle X, Y, $R_1$, $R_2$, $R_3$, $R_4$, Y et Ar sont tels que définis précédemment, et $R_{5b}$ représente un groupement $OR_c$, $NR_cR_d$ dans lesquels $R_c$ et $R_d$ sont tels que définis dans la formule (I),

b) soit à l'action d'un anhydride de formule (XIII) ou d'un chlorure d'acide de formule (XIV):

$(R_{10})_2O$ (XIII) $R_{10}$-Cl (XIV)

dans laquelle $R_{10}$ représente un groupement de formule $C(W_2)$-U-V ou $C(W_2)$-$W_3$-$T_1$ dans lesquelles $W_2$, $W_3$, U, V et $T_1$ sont tels que définis dans la formule (I), pour conduire aux composés de formule (I/f), cas particulier des composés de formule (I) :

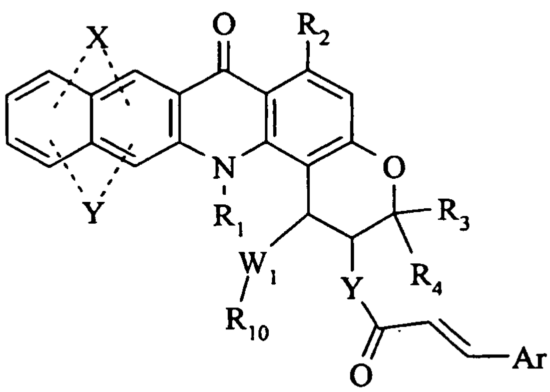

(I/f)

dans laquelle X, Y, $R_1$, $R_2$, $R_3$, $R_4$, Y, Ar et $R_{10}$ sont tels que définis précédemment et $W_1$ est tel que défini dans la formule (I),

les composés (I/a) à (I/f) formant l'ensemble des composés de l'invention qui sont purifiés, le cas échéant, selon une technique classique de purification, qui peuvent, si on le désire, être séparés en leurs différents isomères selon une technique classique de séparation et qui sont transformés, si on le souhaite, en leurs sels d'addition à un acide ou à une base pharmaceutiquement acceptable.

Les composés de formules (II), (V), (IX), (X), (XII), (XIII) et (XIV) sont soit des produits commerciaux, soit obtenus selon des méthodes classiques de la synthèse organique bien connues de l'homme du métier.

Les composés de formule (I) présentent des propriétés antitumorales particulièrement intéressantes. Ils ont une excellente cytotoxicité in vitro sur des lignées cellulaires, issues de tumeurs murines et humaines, due à un blocage spécifique du cycle cellulaire, et sont actifs in vivo, chez la souris, sur des tumeurs transplantables murines et humaines. Les propriétés caractéristiques de ces composés permettent leur utilisation en thérapeutique en tant qu'agents antitumoraux.

La présente invention a également pour objet les compositions pharmaceutiques contenant comme principe actif au moins un composé de formule (I), ses énantiomères, diastéréoisomères ou un de ses sels d'addition à une base

ou un acide pharmaceutiquement acceptable, seul ou en combinaison avec un ou plusieurs excipients ou véhicules inertes, non toxiques, pharmaceutiquement acceptables.

Parmi les compositions pharmaceutiques selon l'invention, il sera cité plus particulièrement celles qui conviennent pour l'administration orale, parentérale (intraveineuse, intramusculaire, ou sous-cutanée), per ou trans-cutanée, intravaginale, rectale, nasale, perlinguale, buccale, oculaire ou respiratoire.

Les compositions pharmaceutiques selon l'invention pour les injections parentérales comprennent notamment les solutions stériles aqueuses et non aqueuses, les dispersions, les suspensions ou émulsions ainsi que les poudres stériles pour la reconstitution des solutions ou des dispersions injectables.

Les compositions pharmaceutiques selon l'invention, pour les administrations orales solides, comprennent notamment les comprimés simples ou dragéifiés, les comprimés sublinguaux, les sachets, les gélules, les granules, et pour les administrations liquides orales, nasales, buccales ou oculaires, comprennent notamment les émulsions, les solutions, les suspensions, les gouttes, les sirops et les aérosols.

Les compositions pharmaceutiques pour l'administration rectale ou vaginale sont préférentiellement des suppositoires, et celles pour l'administration per ou trans-cutanée comprennent notamment les poudres, les aérosols, les crèmes, les pommades, les gels et les patchs.

Les compositions pharmaceutiques citées précédemment illustrent l'invention mais ne la limitent en aucune façon.

Parmi les excipients ou véhicules inertes, non toxiques, pharmaceutiquement acceptables, on peut citer à titre indicatif et non limitatif les diluants, les solvants, les conservateurs, les agents mouillants, les émulsifiants, les agents dispersants, les liants, les agents gonflants, les agents désintégrants, les retardants, les lubrifiants, les absorbants, les agents de suspension, les colorants, les aromatisants, etc...

La posologie utile varie selon l'âge et le poids du patient, la voie d'administration, la composition pharmaceutique utilisée, la nature et la sévérité de l'affection, et la prise de traitements associés éventuels. La posologie s'échelonne de 0,1 mg à 1000 mg en une ou plusieurs prises par jour.

Les exemples suivants illustrent l'invention mais ne la limitent en aucune façon.

Les produits de départ utilisés sont des produits connus ou préparés selon des modes opératoires connus. Les différentes préparations conduisent à des intermédiaires de synthèse utiles pour la préparation des composés de l'invention.

Les structures des composés décrits dans les exemples et dans les préparations ont été déterminées selon les techniques spectrophotométriques usuelles (infrarouge, résonance magnétique nucléaire, spectrométrie de masse, ...).

Les points de fusion ont été déterminés soit à la platine chauffante de Kofler, soit à la platine chauffante sous microscope. Lorsque le composé existe sous forme de sel, le point de fusion correspond à celui du produit salifié.

**PREPARATION 1 : Chlorure de 4-chlorocinnamoyle**

A une suspension de 1,75 g d'acide 4-chlorocinnamique dans 40 ml de dichlorométhane anhydre, sous agitation à 43°C, sont ajoutés goutte à goutte 7 ml de chlorure de thionyle. Après 3 heures de réaction, l'excès d'acide qui n'a pas réagi reste insoluble et est éliminé par décantation. Le filtrat est évaporé à sec fournissant le chlorure de l'acide 4-chloracinnamique.

**PREPARATION 2 : Chlorure de 2-chlorocinnamoyle**

Le produit est obtenu selon le procédé de la préparation 1 en utilisant l'acide 2-chlorocinnamique à la place de l'acide 4-chlorocinnamique.

**PREPARATION 3 : Chlorure de 3-chlorocinnamoyle**

Le produit est obtenu selon le procédé de la préparation 1 en utilisant l'acide 3-chlorocinnamique à la place de l'acide 4-chlorocinnamique.

**PREPARATION 4 : Chlorure de 2,4-dichlorocinnamoyle**

Le produit est obtenu selon le procédé de la préparation 1 en utilisant l'acide 2,4-dichloro-cinnamique à la place de l'acide 4-chlorocinnamique.

**PREPARATION 5 : Chlorure de 3,4-dichlorocinnamoyle**

Le produit est obtenu selon le procédé de la préparation 1 en utilisant l'acide 3,4-dichloro-cinnamique à la place de l'acide 4-chlorocinnamique.

**PREPARATION 6 : Chlorure de 4-bromocinnamoyle**

Le produit est obtenu selon le procédé de la préparation 1 en utilisant l'acide 4-bromo-cinnamique à la place de l'acide 4-chlorocinnamique.

**PREPARATION 7 : Chlorure de 4-méthoxycinnamoyle**

Le produit est obtenu selon le procédé de la préparation 1 en utilisant l'acide 4-méthoxy-cinnamique à la place de l'acide 4-chlorocinnamique.

**PREPARATION 8 : Chlorure de 4-nitrocinnamoyle**

Le produit est obtenu selon le procédé de la préparation 1 en utilisant l'acide 4-nitrocinnamique à la place de l'acide 4-chlorocinnamique.

**PREPARATION 9 : Chlorure de 4-fluorocinnamoyle**

Le produit est obtenu selon le procédé de la préparation 1 en utilisant l'acide 4-fluorocinnamique à la place de l'acide 4-chlorocinnamique.

**PREPARATION 10 : Chlorure de 3,4-diméthoxycinnamoyle**

Le produit est obtenu selon le procédé de la préparation 1 en utilisant l'acide 3,4-diméthoxycinnamique à la place de l'acide 4-chlorocinnamique.

**PREPARATION 11 : Chlorure de 3-trifluorométhylcinnamoyle**

Le produit est obtenu selon le procédé de la préparation 1 en utilisant l'acide 3-trifluorométhylcinnamique à la place de l'acide 4-chlorocinnamique.

**PREPARATION 12 : Chlorure de 3-bromocinnamoyle**

Le produit est obtenu selon le procédé de la préparation 1 en utilisant l'acide 3-bromocinnamique à la place de l'acide 4-chlorocinnamique.

**PREPARATION 13 : Chlorure de 4-trifluorométhylcinnamoyle**

Le produit est obtenu selon le procédé de la préparation 1 en utilisant l'acide 4-trifluorométhylcinnamique à la place de l'acide 4-chlorocinnamique.

**PREPARATION 14 : Chlorure de 3-(1-naphthyl)acryloyle**

Le produit est obtenu selon le procédé de la préparation 1 en utilisant l'acide 3-(1-naphthyl)acrylique à la place de l'acide 4-chlorocinnamique.

**PREPARATION 15 : Chlorure de 3-(2-naphthyl)acryloyle**

Le produit est obtenu selon le procédé de la préparation 1 en utilisant l'acide 3-(2-naphthyl)acrylique à la place de l'acide 4-chlorocinnamique.

**EXEMPLE 1 : (±)-*cis*-2-Cinnamoyioxy-1-hydroxy-6-méthoxy-3,3,14-triméthyl-1,2,3,14-tétrahydro-1*H*-benzo[*b*] pyrano[3,2-*h*]acridin-7-one**

A une solution de 520 mg de (±)cis-1,2-dihydroxy-6-méthoxy-3,3,14-triméthyl-2,3,7,14-tétrahydro-1*H*-benzo [*b*]pyrano[3,2-*h*]acridin-6-one (EP 1 042 326) dans 15 ml de pyridine anhydre, sont additionnés 619 mg de chlorure de cinnamoyle. Après 48 heures sous agitation à 20°C, le solvant est évaporé à sec sous pression réduite à une température ne dépassant pas 20°C. Une chromatographie sur gel de silice (dichlorométhane puis gradient de méthanol de 0,2 à 1 %) suivie d'une précipitation dans l'éthanol permet d'isoler 233 mg du produit attendu.
*Spectrométrie de masse (DIC/$NH_3$) : m/z = 536 $[MH]^+$*

**EXEMPLE 2 : (±)-*cis*-1-Acétoxy-2-cinnamoyloxy-6-méthoxy-3,3,14-triméthyl-1,2,3,14-tétrahydro-1*H*-benzo[*b*] pyrano[3,2-*h*]acridin-7-one**

A une solution de 170 mg du composé de l'exemple 1 dans 5 ml de pyridine anhydre, sont additionnés 3 ml d'anhydride acétique et 7 mg de 4-diméthylaminopyridine. Le milieu réactionnel est maintenu sous agitation à température ambiante pendant 5 heures et est ensuite versé sur 20 ml d'eau glacée. Le précipité obtenu est filtré, lavé à l'eau, séché sous vide phosphorique permettant d'isoler 130 mg du produit attendu.
*Spectrométrie de masse ($ES^+$) : m/z = 5 78 $[MH]^+$*

**EXEMPLE 3 : (±)-*cis*-2-(4-Chlorocinnamoyloxy)-1-hydroxy-6-méthoxy-3,3,14-triméthyl-1,2,3,14-tétrahydro-1*H*-benzo[*b*]pyrano[3,2-*h*]acridin-7-one**

Le produit est obtenu selon le procédé de l'exemple 1 en utilisant le composé de la préparation 1 à la place du chlorure de cinnamoyle.
*Spectrométrie de masse ($ES^+$) : m/z = 569 et 571 $[MH]^+$*

**EXEMPLE 4 : (±)-*cis*-1-Acétoxy-2-(4-chlorocinnamoyloxy)-6-méthoxy-3,3,14-triméthyl-1,2,3,14-tétrahydro-1*H*-benzo[*b*]pyrano[3,2-*h*]acridin-7-one**

Le produit est obtenu selon le procédé de l'exemple 2 en utilisant le composé de l'exemple 3.
*Spectrométrie de masse ($ES^+$) : m/z = 612 et 614 $[MH]^+$*

**EXEMPLE 5 : (±)-*cis*-2-(2-Chlorocinnamoyloxy)-1-hydroxy-6-méthoxy-3,3,14-triméthyl-1,2,3,14-tétrahydro-1*H*-benzo[*b*]pyrano[3,2-*h*]acridin-7-one**

Le produit est obtenu selon le procédé de l'exemple 1 en utilisant le composé de la préparation 2 à la place du chlorure de cinnamoyle.
*Spectrométrie de masse ($ES^+$) : m/z = 570 et 572 $[MH]^+$*

**EXEMPLE 6 : (±)-*cis*-1-Acétoxy-2-(2-chlorocinnamoyloxy)-6-méthoxy-3,3,14-triméthyl-1,2,3,14-tétrahydro-1*H*-benzo[b]pyrano[3,2-*h*]acridin-7-one**

Le produit est obtenu selon le procédé de l'exemple 2 en utilisant le composé de l'exemple 5.
*Spectrométrie de masse ($ES^+$) : m/z = 612 et 614 $[MH]^+$*

**EXEMPLE 7 : (±)-*cis*-2-(3-Chlorocinnamoyloxy)-1-hydroxy-6-méthoxy-3,3,14-triméthyl-1,2,3,14-tétrahydro-1*H*-benzo[*b*]pyrano[3,2-*h*]acridin-7-one**

Le produit est obtenu selon le procédé de l'exemple 1 en utilisant le composé de la préparation 3 à la place du chlorure de cinnamoyle.
*Spectrométrie de masse ($ES^+$) : m/z = 570 et 572 $[MH]^+$*

**EXEMPLE 8 : (±)-*cis*-1-Acétoxy-2-(3-ehlorocinnamoyloxy)-6-méthoxy-3,3,14-triméthyl-1,2,3,14-tétrahydro-1*H*-benzo[*b*]pyrano[3,2-*h*]acridin-7-one**

Le produit est obtenu selon le procédé de l'exemple 2 en utilisant le composé de l'exemple 7.
*Spectrométrie de masse ($ES^+$) : m/z = 612 et 614 $[MH]^+$*

**EXEMPLE 9 : (±)-*cis*-2-(2,4-Dichlorocinnamoyloxy)-1-hydroxy-6-méthoxy-3,3,14-triméthyl-1,2,3,14-tétrabydro-1*H*-benzo[*b*]pyrano[3,2-*h*]acridin-7-one**

Le produit est obtenu selon le procédé de l'exemple 1 en utilisant le composé de la préparation 4 à la place du chlorure de cinnamoyle.
*Spectrométrie de masse (ES+): mlz = 604, 606 et 608 [MH]+*

**EXEMPLE 10 : (±)-*cis*-1-Acétoxy-2-(2,4-dichlorocinnamoyloxy)-6-méthoxy-3,3,14-triméthyl-1,2,3,14-tétrahydro-1*H*-benzo[*b*]pyrano[3,2-*h*]acridin-7-one**

Le produit est obtenu selon le procédé de l'exemple 2 en utilisant le composé de l'exemple 9.
*Spectrométrie de masse (ES+) : m/z = 646, 648 et 650 [MH]+*

**EXEMPLE 11 : (±)-*cis*-2-(3,4-Dichlorocinnamoyloxy)-1-hydroxy-6-méthoxy-3,3,14-triméthyl-1,2,3,14-tétrahydro-1*H*-benzo[*b*]pyrano[3,2-*h*]acridin-7-one**

Le produit est obtenu selon le procédé de l'exemple 1 en utilisant le composé de la préparation 5 à la place du chlorure de cinnamoyle.
*Spectrométrie de masse (ES+) : m/z = 604, 606 et 608 [MH]+*

**EXEMPLE 12 : (±)-cis-1-Acétoxy-2-(3,4-dichlorocinnamoyloxy)-6-méthoxv-3,3,14-triméthyl-1,2,3,14-tétrahydro-1*H*-benzo[*b*]pyrano[3,2-*h*]acridin-7-one**

Le produit est obtenu selon le procédé de l'exemple 2 en utilisant le composé de l'exemple 11.
*Spectrométrie de masse (ES+) : m/z = 646, 648 et 650 [MH]+*

**EXEMPLE 13 : (±)-*cis*-2-(4-Bromocinnamoyioxy)-1-hydroxy-6-méthoxy-3,3,14-triméthyl-1,2,3,14-tétrahydro-1*H*-benzo[*b*]-pyrano[3,2-*h*]acridin-7-one**

Le produit est obtenu selon le procédé de l'exemple 1 en utilisant le composé de la préparation 6 à la place du chlorure de cinnamoyle.
*Spectrométrie de masse (ES+): mlz = 614 et 616 [MH]+*

**EXEMPLE 14 : (±)-*cis*-1-Acétoxy-2-(4-bromocinnamoyloxy)-6-méthoxy-3,3,14-triméthyl-1,2,3,14-tétrahydro-1*H*-benzo[*b*]pyrano[3,2-*h*]acridin-7-one**

Le produit est obtenu selon le procédé de l'exemple 2 en utilisant le composé de l'exemple 13.
*Spectrométrie de masse (ES+) : m/z = 656 et 658 [MH]+*

**EXEMPLE 15 : (±)-cis-1-Hydroxy-6-méthoxy-2-(4-méthoxycinnamoyloxy)-3,3,14-triméthyl-1,2,3,14-tétrahvdro-1*H*-benzo[*b*]pyrano[3,2-*h*]acridin-7-one**

Le produit est obtenu selon le procédé de l'exemple 1 en utilisant le composé de la préparation 7 à la place du chlorure de cinnamoyle.
*Spectrométrie de masse (ES+) : m/z = 566 [MH]+*

**EXEMPLE 16 : (±)-*cis*-1-Acétoxy-6-méthoxy-2-(4-méthoxycinnamoyloxy)-3,3,14-triméthyl-1,2,3,14-tétrahydro-1*H*-benzo[*b*]pyrano[3.2-*h*]acridin-7-one**

Le produit est obtenu selon le procédé de l'exemple 2 en utilisant le composé de l'exemple 15.
*Spectrométrie de masse (ES+) : m/z = 608 [MH]+*

**EXEMPLE 17 : (±)-*cis*-6-Méthoxy-1,2-di-(4-méthoxycinnamoyloxy)-3,3,14-triméthyl-1,2,3,14-tétrahydro-1*H*-benzo[*b*]pyrano[3,2-*h*]acridin-7-one**

Le produit est obtenu au cours du procédé décrit dans l'exemple 15.
*Spectrométrie de masse (ES+) : m/z = 726 [MH]+*

**EXEMPLE 18 : (±)-*cis*-1-Hydroxy-6-méthoxy-2-(4-nitrocinnamoyloxy)-3,3,14-triméthyl-1,2,3,14-tétrahydro-1*H*-benzo[*b*]pyrano[3,2-*h*]acridin-7-one**

Le produit est obtenu selon le procédé de l'exemple 1 en utilisant le composé de la préparation 8 à la place du chlorure de cinnamoyle.
*Spectrométrie de masse* ($ES^+$) : m/z = 581 $[MH]^+$

**EXEMPLE 19 : (±)-*cis*-1-Acétoxy-6-méthoxy-2-(4-nitrocinnamoyloxy)-3,3,14-triméthyl-1,2,3,14-tétrahydro-1*H*-benzo[*b*]pyrano[3,2-*h*]acridin-7-one**

Le produit est obtenu selon le procédé de l'exemple 2 en utilisant le composé de l'exemple 18.
*Spectrométrie de masse* ($ES^+$): m/z = 623 $[MH]^+$

**EXEMPLE 20 : (±)-*cis*-1,2-Di-(4-fluorocinnamoyloxyl)-6-méthoxy-3,3,14-triméthyl-1,2,3,14-tétrahydro-1*H*-benzo[*b*]pyrano[3,2-*h*]acridin-7-one**

Le produit est obtenu selon le procédé de l'exemple 1 en utilisant le composé de la préparation 9 à la place du chlorure de cinnamoyle.
*Spectrométrie de masse* ($ES^+$) : m/z = 703 $[MH]^+$

**EXEMPLE 21 : (±)-*cis*-2-(3,4-Diméthoxycinnamoyloxy)-1-hydroxy-6-méthoxy-3,3,14-triméthyl-1,2,3,14-tétrahydro-7*H*-benzo[*b*]pyrano[3,2-*h*]acridin-7-one**

Le produit est obtenu selon le procédé de l'exemple 1 en utilisant le composé de la préparation 10 à la place du chlorure de cinnamoyle.
*Spectrométrie de masse* ($ES^+$): m/z = 596 $[MH]^+$

**EXEMPLE 22 : (±)-*cis*-1-Acétoxy-2-(3,4-diméthoxycinnamoyloxy)-6-méthoxy-3,3,14-triméthyl-1,2,3,14-tétrahydro-7*H*-benzo[*b*]pyrano[3,2-*h*]acridin-7-one**

Le produit est obtenu selon le procédé de l'exemple 2 en utilisant le composé de l'exemple 21.
*Spectrométrie de masse* ($ES^+$) : m/z = 638 $[MH]^+$

**EXEMPLE 23 : (±)-*cis*-1-Hydroxy-6-méthoxy-2-(3-trifluorométhyleinnamoyloxy)-3,3, 14-triméthyl-1,2,3,14-tétrahydro-7*H*-benzo[*b*]pyrano[3,2-*h*]acridin-7-one**

Le produit est obtenu selon le procédé de l'exemple 1 en utilisant le composé de la préparation 11 à la place du chlorure de cinnamoyle.
*Spectrométrie de masse* ($ES^+$) : m/z = 604, 605 $[MH]^+$

**EXEMPLE 24 : (±)-*cis*-1-Acétoxy-6-méthoxy-2-(3-trifluorométhylcinnamoyloxy)-3,3, 14-triméthyl-1,2,3,14-tétrahydro-7*H*-benzo[*b*]pyrano[3,2-*h*]acridin-7-one**

Le produit est obtenu selon le procédé de l'exemple 2 en utilisant le composé de l'exemple 23.
*Spectrométrie de masse* ($ES^+$) : m/z = 646, 647 $[MH]^+$

**EXEMPLE 25 : (±)-*cis*-2-(3-Bromocinnamoyloxy)-1-hydroxy-6-méthoxy-3,3,14-triméthyl-1,2,3,14-tétrahydro-7*H*-benzo[*b*]pyrano[3,2-*h*]acridin-7-one**

Le produit est obtenu selon le procédé de l'exemple 1 en utilisant le composé de la préparation 12 à la place du chlorure de cinnamoyle.
*Spectrométrie de masse* ($ES^+$) : m/z = 614, 616 $[MH]^+$; 636, 638 $[MNa]^+$

**EXEMPLE 26 : (±)-*cis*-1-Acétoxy-2-(3-bromocinnamoyloxy)-6-méthoxy-3,3,14-triméthyl-1,2,3,14-tétrahydro-7*H*-benzo[*b*]pyrano[3,2-*h*]acridin-7-one**

Le produit est obtenu selon le procédé de l'exemple 2 en utilisant le composé de l'exemple 25.
*Spectrométrie de masse* ($ES^+$) : m/z = 656, 658 $[MH]^+$

**EXEMPLE 27 : (±)-*cis*-1-Hydroxy-6-méthoxy-2-(4-trifluorométhyleinnamoyloxy)-3,3, 14-triméthyl-1,2,3,14-tétrahydro-7*H*-benzo[*b*]pyrano[3,2-*h*]acridin-7-one**

Le produit est obtenu selon le procédé de l'exemple 1 en utilisant le composé de la préparation 13 à la place du chlorure de cinnamoyle.
*Spectrométrie de masse (ES$^+$) : m/z = 604, 605 [MH]$^+$*

**EXEMPLE 28 : (±)-*cis*-1-Acétoxy-6-méthoxy-2-(4-trifluorométhylcinnamoyloxy)-3,3, 14-triméthyl-1,2,3,14-tétrahydro-7*H*-benzo[*b*]pyrano[3,2-*h*]acridin-7-one**

Le produit est obtenu selon le procédé de l'exemple 2 en utilisant le composé de l'exemple 27.
*Spectrométrie de masse (ES$^+$) : m/z = 646, 647 [MH]$^+$*

**EXEMPLE 29 : (±)-*cis*-1-Hydroxy-6-méthoxy-2-(3-(1-naphthyl)-acryloyloxy)-3,3, 14-triméthyl-1,2,3,14-tétrahydro-7*H*-benzo[*b*]pyrano[3,2-*h*]acridin-7-one**

Le produit est obtenu selon le procédé de l'exemple 1 en utilisant le composé de la préparation 14 à la place du chlorure de cinnamoyle.
*Spectrométrie de masse (ES$^+$): m/z = 586 [MH]$^+$*

**EXEMPLE 30 : (±)-*cis*-1-Acétoxy-6-méthoxy-2-(3-1-naphthylacryloyloxy)-3,3, 14-triméthyl-1,2,3,14-tétrahydro-7*H*-benzo[*b*]pyrano[3,2-*h*]acridin-7-one**

Le produit est obtenu selon le procédé de l'exemple 2 en utilisant le composé de l'exemple 29.
*Spectrométrie de masse (ES$^+$) : m/z = 628 [MH]$^+$*

**EXEMPLE 31 : (±)-*cis*-1-Hydroxy-6-méthoxy-2-(3-(2-naphthyl)acryloyloxy)-3,3, 14-triméthyl-1,2,3,14-tétrahydro-7*H*-benzo[*b*]pyrano[3,2-*h*]acridin-7-one**

Le produit est obtenu selon le procédé de l'exemple 1 en utilisant le composé de la préparation 15 à la place du chlorure de cinnamoyle.
*Spectrométrie de masse (ES$^+$) : m/z = 586 [MH]$^+$*

**EXEMPLE 32 : (±)-*cis*-1-Acétoxy-2-(3,4-dichlorocinnamoyloxy)-6-méthoxy-3,3,14-triméthyl-1,2,3,14-tétrahydro-1*H*-benzo[*b*]pyrano[3,2-*h*]acridin-7-one (énantiomère α)**

L'énantiomère α du composé de l'exemple 12 est obtenu par séparation chirale sur colonne chiralcel oc.

**EXEMPLE 33 : (±)-*cis*-1-Acétoxy-2-(3,4-dichlorocinnamoyloxy)-6-méthoxy-3,3,14-triméthyl-1,2,3,14-tétrahydro-1*H*-benzo[*b*]pyrano[3,2-*h*]acridin-7-one (énantiomère β)**

L'énantiomère P du composé de l'exemple 12 est obtenu par séparation chirale sur colonne chiralcel oc.

***ETUDE PHARMACOLOGIQUE DES COMPOSES DE L'INVENTION***

**EXEMPLE A : Cytotoxicité in vitro**

Deux lignées cellulaires ont été utilisées :

- 1 leucémie murine, L1210,
- 1 carcinome épidermoïde humain : KB-3-1

Les cellules sont cultivées dans du milieu RPMI 1640 complet contenant 10 % de sérum de veau foetal, 2 mM de glutamine, 50 unités/ml de pénicilline, 50 μg/ml de streptomycine et 10 mM d'Hepes, pH = 7,4. Les cellules sont réparties dans des microplaques et exposées aux composés cytotoxiques. Les cellules sont ensuite incubées pendant 2 jours (L1210) ou 4 jours (KB-3-1) dans un incubateur, à 37°C en présence de 5 % de $CO_2$. Le nombre de cellules viables est ensuite quantifié par un essai colorimétrique, le Microculture Tetrazolium Assay (Cancer Res. 1987, 47, 939-942).

Les résultats sont exprimés en $IC_{50}$, concentration en cytotoxique qui inhibe à 50 % la prolifération des cellules traitées. A titre d'exemple, le composé de l'exemple 2 présente respectivement une $IC_{50}$ de 0,59 μM sur L1210 et de 0,151 μM sur KB-3-1.

**EXEMPLE B : Activité in vivo**

***Activité antitumorale sur l'adénocarcinome du colon C38***

Les fragments tumoraux d'adénocarcinome du colon C38, pesant environ 30 mg, ont été implantés au jour 0 sous la peau de souris B6D2F1 (Iffa Credo, France).
Après croissance de la tumeur, les souris ont été séparées en groupes contrôle (18 animaux) et traité (6 à 7 animaux), homogènes quant à la taille tumorale. Les produits ont été administrés par voie i.v. une fois par semaine pendant 3 semaines (aux jours 10, 17 et 24), à leur Dose Maximale Tolérée (MTD), MTD/2 et MTD/4.
Les tumeurs ont été mesurées deux fois par semaine et les volumes tumoraux ont été calculés suivant la formule : Volume ($mm^3$) = longueur (mm) x largeur ($mm^2$) / 2.
L'activité antitumorale est exprimée en % de T/C :

$$\% \ T/C = \frac{Vt/V0 \text{ médian des animaux traités}}{Vt/V0 \text{ médian des animaux contrôle}} \times 100$$

VO et Vt étant respectivement le volume initial de la tumeur et son volume au temps de mesure t.
La dose optimale est la dose qui donne le T/C le plus bas sans toxicité (mort prématurée ou perte de poids supérieure à 20%).
A titre d'exemple, les composés de l'exemple 33 montre une activité antitumorale de 50 % pour une dose optimale de 4 mg/kg alors que l'acronycine montre une activité antitumorale de 27 % pour une dose optimale de 100 mg/kg permettant de démontrer leur fort potentiel thérapeutique.

**EXEMPLE C : Composition pharmaceutique**

| Formule de préparation pour 1000 comprimés dosés à 10 mg | |
|---|---|
| Composé de l'exemple 2 | 10 g |
| Hydroxypropylcellulose | 2 g |
| Amidon de blé | 10 g |
| Lactose | 100 g |
| Stéarate de magnésium | 3 g |
| Talc | 3 g |

**Revendications**

1. Composés de formule (I) :

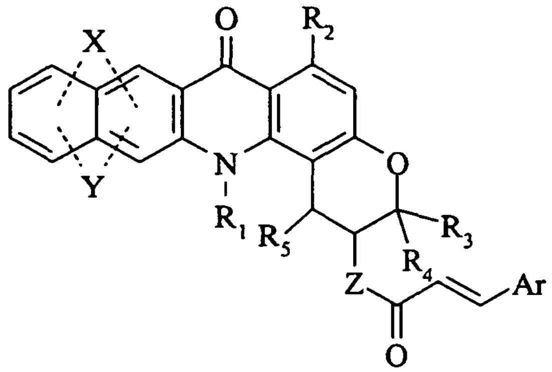

(I)

dans laquelle :

- X et Y, identiques ou différents, indépendamment l'un de l'autre, représentent chacun un groupement choisi parmi :

  - atome d'hydrogène, halogène,
  - groupement hydroxy, alkoxy ($C_1$-$C_6$) linéaire ou ramifié, nitro, cyano, alkyle ($C_1$-$C_6$) linéaire ou ramifié, éventuellement substitué par un ou plusieurs groupements choisis parmi hydroxy et halogène et alkényle ($C_2$-$C_6$) linéaire ou ramifié, ou
  - groupement de formule $-NR_aR_b$ dans lequel :

    $R_a$ et $R_b$, identiques ou différents, indépendamment l'un de l'autre, représentent chacun un groupement choisi parmi atome d'hydrogène, ou groupement alkyle ($C_1$-$C_6$) linéaire ou ramifié,

  ou $R_a$ et $R_b$ forment ensemble avec l'atome d'azote qui les portent, un hétérocycle de 5 à 7 chaînons monocyclique, contenant éventuellement au sein du système cyclique un second hétéroatome choisi parmi oxygène et azote,
  étant entendu que les substituants X et Y peuvent être présents, indépendamment l'un de l'autre, sur l'un ou l'autre des deux cycles benzéniques adjacents,

- Z représente un atome d'oxygène ou $NR_c$ dans lequel $R_c$, représente un groupement choisi parmi atome d'hydrogène, groupement alkyle ($C_1$-$C_6$) linéaire ou ramifié, ou un groupement aryle, arylalkyle ($C_1$-$C_6$) linéaire ou ramifié,
- Ar représente un groupement aryle ou hétéroaryle,
- $R_1$ représente un atome d'hydrogène ou un groupement alkyle ($C_1$-$C_6$) linéaire ou ramifié,
- $R_2$ représente un groupement choisi parmi atome d'hydrogène, groupement alkyle ($C_1$-$C_6$) linéaire ou ramifié, $-OR_a$ ; $-NR_aR_b$ dans lesquels $R_a$ et $R_b$ sont tels que définis précédemment,
- $R_3$, $R_4$, identiques ou différents, indépendamment l'un de l'autre, représentent un atome d'hydrogène ou un groupement alkyle ($C_1$-$C_6$) linéaire ou ramifié,
- $R_5$ représente un groupement choisi parmi :

  1) atome d'hydrogène,
  2) groupement $OR_c$, $NR_cR_d$ dans lesquels :

     $R_c$, est tel que défini précédemment et $R_d$, a les mêmes définitions que $R_c$,

  3) $W_1$-C($W_2$)-U-V dans lequel :

     α) $W_1$ représente un atome d'oxygène ou $NR_c$ (dans lequel $R_c$ est tel que défini précédemment),
     β) $W_2$ représente un atome d'oxygène,
     γ) U représente une liaison simple ou une chaîne alkylène ($C_1$-$C_8$) linéaire ou ramifiée ou une chaîne alkénylène ($C_2$-$C_8$) linéaire ou ramifiée,
     δ) V représente un groupement choisi parmi :

- atome d'hydrogène,
- groupement aryle, hétéroaryle,
- groupements $OR_c$, $CO_2R_c$, $COR_c$, $CONR'_aR'_b$, $NR'_aR'_b$, $N(R_c)$-$CO_2R'_c$, $N(R_c)$-$COR'_c$, dans lesquels $R_c$ est tel que défini précédemment, $R'_c$ a les mêmes définitions que $R_c$ et, $R'_a$ et $R'_b$, identiques ou différents, indépendamment l'un de l'autre, représentent chacun un groupement choisi parmi atome d'hydrogène, groupement alkyle ($C_1$-$C_6$) linéaire ou ramifié, aryle, arylalkyle ($C_1$-$C_6$) linéaire ou ramifié, ou $R'_a$ et $R'_b$ forment ensemble avec l'atome d'azote qui les portent, un hétérocycle de 5 à 7 chaînons, monocyclique, contenant éventuellement au sein du système cyclique un second hétéroatome choisi parmi oxygène et azote,

4) $W_1$-$C(W_2)$-$W_3$-$T_1$ dans lequel :

α) $W_1$ et $W_2$ sont tels que définis précédemment,
β) $W_3$ représente un atome d'oxygène ou $NR_c$ dans lequel $R_c$ est tel que défini précédemment,
γ) $T_1$ représente un groupement choisi parmi :

- atome d'hydrogène,
- alkyle ($C_1$-$C_6$) linéaire ou ramifié,
- alkényle ($C_2$-$C_6$) linéaire ou ramifié,
- aryle, arylalkyle ($C_1$-$C_6$) linéaire ou ramifié,
- chaîne alkylène ($C_1$-$C_6$) linéaire ou ramifiée ou une chaîne alkénylène ($C_2$-$C_6$) linéaire ou ramifiée, chacune étant substituée par un groupement $OR_c$ dans lequel $R_c$ est tel que défini précédemment ou par $NR'_aR'_b$ dans lequel $R'_a$ et $R'_b$ sont tels que définis précédemment,

5) Z-CO-CH=CHAr dans lequel Z et Ar sont tels que définis précédemment,

leurs énantiomères, diastéréoisomères, lorsqu'ils existent, ainsi que leurs sels d'addition à un acide ou à une base pharmaceutiquement acceptable, ainsi que leurs hydrates et leurs solvats,
étant entendu que :

par aryle, on comprend un groupement phényle ou naphtyle, comportant éventuellement un ou plusieurs substituants, identiques ou différents, choisis parmi alkyle ($C_1$-$C_6$) linéaire ou ramifié éventuellement substitué par un ou plusieurs groupements hydroxy ou halogène, hydroxy, halogène, carboxy, nitro, amino, monoalkylamino($C_1$-$C_6$)linéaire ou ramifié, ou dialkylamino($C_1$-$C_6$)linéaire ou ramifié, alkoxy ($C_1$-$C_6$) linéaire ou ramifié, acyle ($C_1$-$C_6$) linéaire ou ramifié, et alkylcarbonyloxy ($C_1$-$C_6$) linéaire ou ramifié,
par hétéroaryle, on entend un groupement de 5 à 12 chaînons, soit monocyclique aromatique, soit bicyclique dont l'un au moins des cycles possède un caractère aromatique, et contenant un, deux ou trois hétéroatomes choisis parmi oxygène, azote ou soufre, étant entendu que l'hétéroaryle peut être éventuellement substitué par un ou plusieurs atomes ou groupements, identiques ou différents, choisis parmi les atomes d'halogène et les groupements alkyle ($C_1$-$C_6$) linéaire ou ramifié, éventuellement substitué par un ou plusieurs groupements hydroxy ou halogène, ou hydroxy, alkoxy ($C_1$-$C_6$) linéaire ou ramifié, ou amino (substitué éventuellement par un ou deux groupements alkyle ($C_1$-$C_6$) linéaire ou ramifié).

**2.** Composés de formule (I) selon la revendication 1, **caractérisés en ce que** X et Y représentent un atome d'hydrogène, leurs énantiomères, diastéréoisomères ainsi que leurs sels d'addition à un acide ou à une base pharmaceutiquement acceptable.

**3.** Composés de formule (I) selon l'une quelconque des revendications 1 à 2, **caractérisés en ce que** $R_1$, $R_3$ et $R_4$ représentent un groupement alkyle ($C_1$-$C_6$) linéaire ou ramifié, leurs énantiomères, diastéréoisomères ainsi que leurs sels d'addition à un acide ou à une base pharmaceutiquement acceptable.

**4.** Composés de formule (I) selon l'une quelconque des revendications 1 à 3, **caractérisés en ce que** $R_2$ représente le groupement $-OR_a$ dans lequel $R_a$ est tel que défini dans la formule (I), leurs énantiomères, diastéréoisomères ainsi que leurs sels d'addition à un acide ou à une base pharmaceutiquement acceptable.

**5.** Composés de formule (I) selon l'une quelconque des revendications 1 à 4, **caractérisés en ce que** $R_5$ représente les groupements $-OR_c$ ou $W_1$-$C(W_2)$-U-V dans lesquels $R_c$, $W_1$, $W_2$, U et V sont tels que définis dans la formule (I), leurs énantiomères, diastéréoisomères ainsi que leurs sels d'addition à un acide ou à une base pharmaceutiquement

acceptable.

**6.** Composés de formule (I) selon l'une quelconque des revendications 1 à 5, **caractérisés en ce que** $R_5$ représente le groupement $-OR_c$ dans lequel $R_c$ représente un atome d'hydrogène ou $R_5$ représente le groupement $W_1$-C($W_2$)-U-V dans lequel $W_1$ et $W_2$ représentent chacun un atome d'oxygène, U représente une chaîne alkylène ($C_1$-$C_8$) linéaire ou ramifiée et V représente un atome d'hydrogène, leurs énantiomères, diastéréoisomères ainsi que leurs sels d'addition à un acide ou à une base pharmaceutiquement acceptable.

**7.** Composés de formule (I) selon l'une quelconque des revendications 1 à 6, **caractérisés en ce que** Z représente un atome d'oxygène, leurs énantiomères, diastéréoisomères ainsi que leurs sels d'addition à un acide ou à une base pharmaceutiquement acceptable.

**8.** Composés de formule (I) selon l'une quelconque des revendications 1 à 7 **caractérisés en ce que** Ar représente un groupement phényle éventuellement substitué, leurs énantiomères, diastéréoisomères ainsi que leurs sels d'addition à un acide ou à une base pharmaceutiquement acceptable.

**9.** Composés de formule (I) selon la revendication 1 qui sont le :

➢ (±)-*cis*-2-cinnamoyloxy-1-hydroxy-6-méthoxy-3,3,14-triméthyl-1,2,3,14,-tétrahydro-1*H*-benzo[*b*]pyrano[3,2-*h*]acridin-7-one,
➢ (±)-*cis*-1-acétoxy-2-cinnamoyloxy-6-méthoxy-3,3,14-triméthyl-1,2,3,14,-tétrahydro-1*H*-benzo[*b*]pyrano[3,2-*h*]acridin-7-one,
➢ (±)-*cis*-2-(4-chlorocinnamoyloxy)-1-hydroxy-6-méthoxy-3,3,14-triméthyl-1,2,3,14,-tétrahydro-1*H*-benzo[*b*]pyrano[3,2-*h*]acridin-7-one,
➢ (±)-*cis*-1-acétoxy-2-(4-chlorocinnamoyloxy)-6-méthoxy-3,3,14-triméthyl-1,2,3,14,-tétrahydro-1*H*-benzo[*b*]pyrano[3,2-*h*]acridin-7-one,
➢ (±)-*cis*-2-(2-chlorocinnamoyloxy)-1-hydroxy-6-méthoxy-3,3,14-triméthyl-1,2,3,14,-tétrahydro-1*H*-benzo[*b*]pyrano[3,2-*h*]acridin-7-one,
➢ (±)-*cis*-1-acétoxy-2-(2-chlorocinnamoyloxy)-6-méthoxy-3,3,14-triméthyl-1,2,3,14,-tétrahydro-1*H*-benzo[*b*]pyrano[3,2-*h*]acridin-7-one,
➢ (±)-*cis*-2-(3-chlorocinnamoyloxy)-1-hydroxy-6-méthoxy-3,3,14-triméthyl-1,2,3,14,-tétrahydro-1*H*-benzo[*b*]pyrano[3,2-*h*]acridin-7-one,
➢ (±)-*cis*-2-(2,4-dichlorocinnamoyloxy)-1-hydroxy-6-méthoxy-3,3,14-triméthyl-1,2,3,14,-tétrahydro-1*H*-benzo[*b*]pyrano[3,2-*h*]acridin-7-one,
➢ (±)-*cis*-1-acétoxy-2-(2,4-dichlorocinnamoyloxy)-6-méthoxy-3,3,14-triméthyl-1,2,3,14,-tétrahydro-1*H*-benzo[*b*]pyrano[3,2-*h*]acridin-7-one,
➢ (±)-*cis*-2-(3,4dichlorocinnamoyloxy)-1-hydroxy-6-méthoxy-3,3,14-triméthyl-1,2,3,14,-tétrahydro-1*H*-benzo[*b*]pyrano[3,2-*h*]acridin-7-one,
➢ (±)-*cis*-1-acétoxy-2-(3,4-dichlorocinnamoyloxy)-6-méthoxy-3,3,14-triméthyl-1,2,3,14,-tétrahydro-1*H*-benzo[*b*]pyrano[3,2-*h*]acridin-7-one,
➢ (±)-*cis*-2-(4-bromocinnamoyloxy)-1-hydroxy-6-méthoxy-3,3,14-triméthyl-1,2,3,14,-tétrahydro-1*H*-benzo[*b*]pyrano[3,2-*h*]acridin-7-one,
➢ (±)-*cis*-1-acétoxy-2-(4-bromocinnamoyloxy)-6-méthoxy-3,3,14-triméthyl-1,2,3,14,-tétrahydro-1*H*-benzo[*b*]pyrano[3,2-*h*]acridin-7-one,
➢ (±)-*cis*-1-hydroxy-6-méthoxy-2-(4-méthoxycinnamoyloxy)-3,3,14-triméthyl-1,2,3,14,-tétrahydro-1*H*-benzo[*b*]pyrano[3,2-*h*]acridin-7-one,
➢ (±)-*cis*-1-hydroxy-6-méthoxy-2-(4-nitrocinnamoyloxy)-3,3,14-triméthyl-1,2,3,14,-tétrahydro-1*H*-benzo[*b*]pyrano[3,2-*h*]acridin-7-one,
➢ (±)-*cis*-1-acétoxy-6-méthoxy-2-(4-nitrocinnamoyloxy)-3,3,14-triméthyl-1,2,3,14,-tétrahydro-1*H*-benzo[*b*]pyrano[3,2-*h*]acridin-7-one,

leurs énantiomères, diastéréoisomères ainsi que leurs sels d'addition à un acide ou à une base pharmaceutiquement acceptable.

**10.** Procédé de préparation des composés de formule (I) selon la revendication 1 **caractérisé en ce qu'**on utilise comme produit de départ un composé de formule (II) :

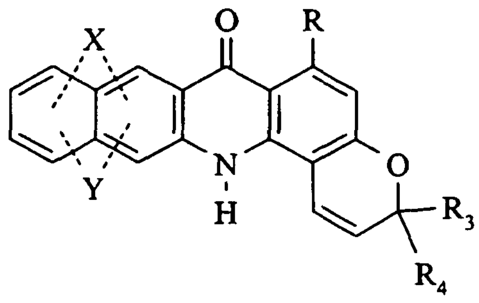

(II)

dans laquelle X, Y, $R_3$ et $R_4$ sont tels que définis précédemment, et R représente un atome d'hydrogène, un groupement hydroxy, un groupement alkyle ($C_1$-$C_6$) linéaire ou ramifié, composé de formule (II) dont l'atome d'azote est substitué ou non, par action d'un halogénure d'alkyle ou d'un sulfate de dialkyle en présence d'un agent de déprotonation, en solvant polaire aprotique ou en condition de transfert de phase, permettant d'obtenir les composés de formule (III) :

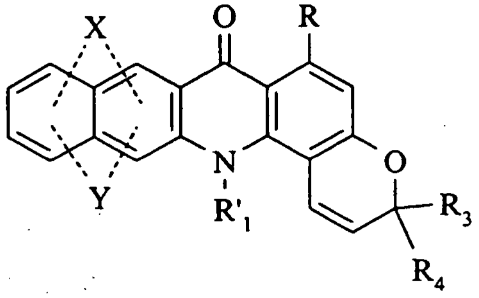

(III)

dans laquelle X, Y, R, $R_3$ et $R_4$ sont tels que définis précédemment, et $R'_1$ représente un groupement alkyle ($C_1$-$C_6$) linéaire ou ramifié,
composés de formule (III) qui sont soumis à l'action d'un agent alkylant selon des conditions classiques de la synthèse organique, pour conduire aux composés de formule (IV):

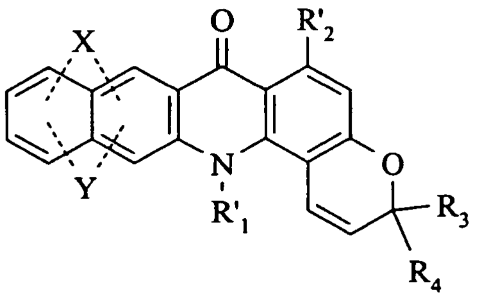

(IV)

dans laquelle X, Y, $R'_1$, $R_3$ et $R_4$ sont tels que définis précédemment et $R'_2$ représente un groupement choisi parmi $OR'_a$ dans lequel $R'_a$ représente un groupement alkyle ($C_1$-$C_6$) linéaire ou ramifié,
composés de formule (IV) qui sont traités, dans le cas où $R'_2$ représente un groupement alkoxy par un composé de formule (V) :

$HNR_aR_b$ (V)

dans laquelle $R_a$ et $R_b$ sont tels que définis dans la formule (I),
pour conduire aux composés de formule (VI) :

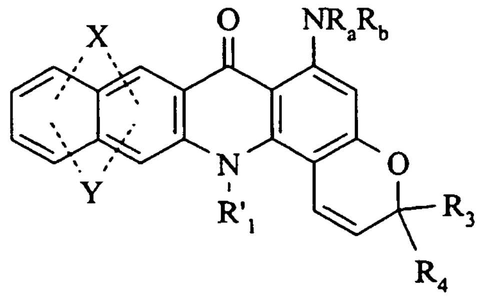

(VI)

dans laquelle X, Y, R'$_1$, $R_3$, $R_4$, $R_a$ et $R_b$ sont tels que définis précédemment, l'ensemble des composés de formule (II), (III), (IV) et (VI) formant les composés de formule (VII) :

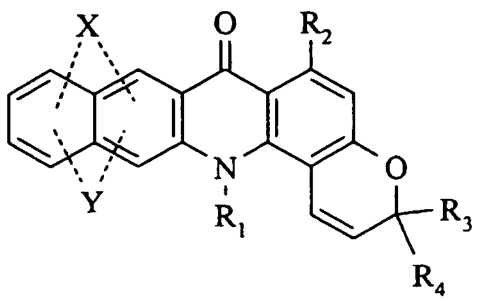

(VII)

dans laquelle X, Y, $R_1$, $R_2$, $R_3$ et $R_4$ sont tels que définis dans la formule (I), composés de formule (VII) qui sont soumis :

a) soit à l'action du tétroxyde d'osmium en milieu polaire et en présence de 4-méthylmorpholine-N-oxyde, pour conduire aux composés de formule (VIII/a) :

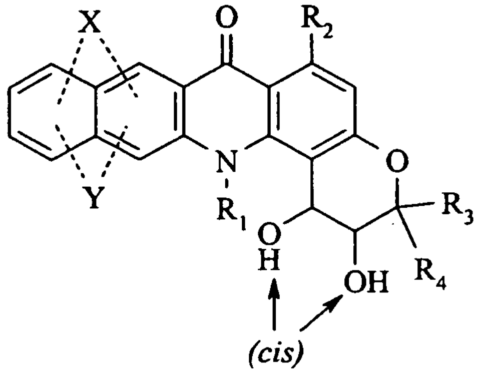

(VIII/a)

dans laquelle X, Y, $R_1$, $R_2$, $R_3$ et $R_4$ sont tels que définis précédemment,

b) soit à l'action de permanganate de potassium en milieu polaire puis à des conditions réductrices en présence de $NaBH_4$, pour conduire au composé de formule (VIII/b) :

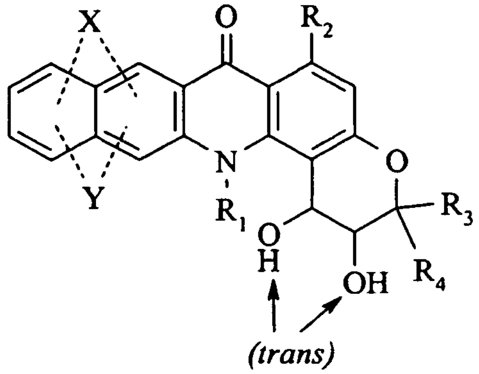


(VIII/b)

dans laquelle X, Y, $R_1$, $R_2$, $R_3$ et $R_4$ sont tels que définis précédemment,
l'ensemble des composés de formules (VIII/a) et (VIII/b) formant les composés de formule (VIII) dont les 2 groupements alcools peuvent être de configuration *cis* ou *trans* l'un par rapport à l'autre :

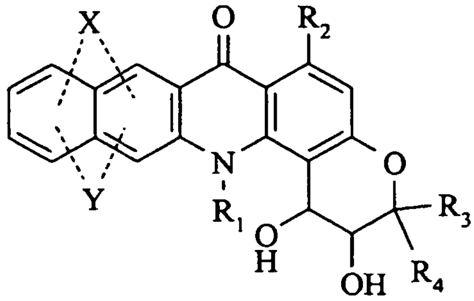

(VIII)

dans laquelle X, Y, $R_1$, $R_2$, $R_3$ et $R_4$ sont tels que définis précédemment,
composés de formule (VIII) qui sont soumis à l'action d'un ou de 2 équivalents d'un anhydride de formule (IX) ou d'un chlorure d'acide de formule (X) :

$$[Ar\text{-}CH\text{=}CH\text{-}C(O)]_2O \quad (IX) \quad Ar\text{-}HC\text{=}CH\text{-}C(O)\text{-}Cl \qquad (X)$$

dans lesquelles Ar est tel que défini dans la formule (I) pour conduire aux composés de formules ($1/a_1$) ou ($I/a_2$), cas particuliers des composés de formule (I) :

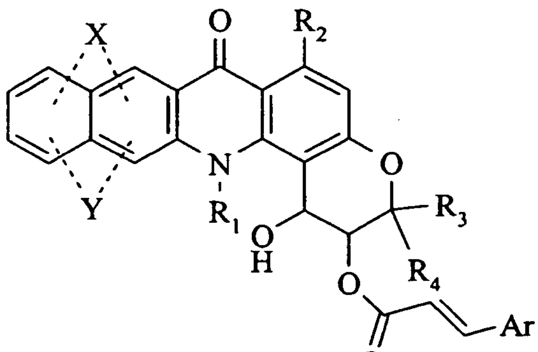

($I/a_1$)

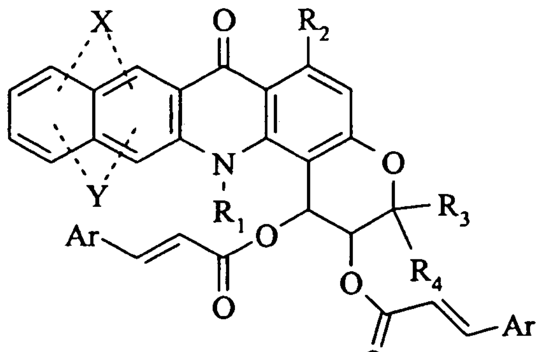

($I/a_2$)

dans lesquelles X, Y, $R_1$, $R_2$, $R_3$, $R_4$ et Ar sont tels que définis précédemment,
c) soit à l'action de $NaN_3$, en présence d'eau oxygénée, suivi d'une étape de réduction, pour conduire aux composés de formule (XI) :

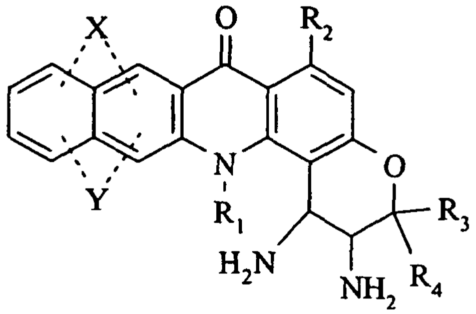

(XI)

dans laquelle X, Y, $R_1$, $R_2$, $R_3$ et $R_4$ sont tels que définis précédemment, composés de formule (XI) qui sont soumis à l'action des composés de formules (IX) ou (X) tels que définis précédemment dans les mêmes conditions que pour les composés de formule (VIII), pour conduire aux composés de formules ($I/b_1$) ou ($I/b_2$), cas particuliers des composés de formule (I) :

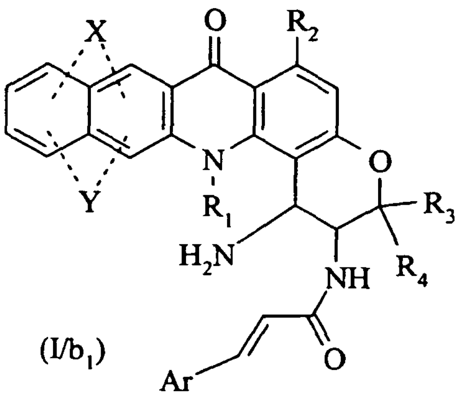

($I/b_1$)

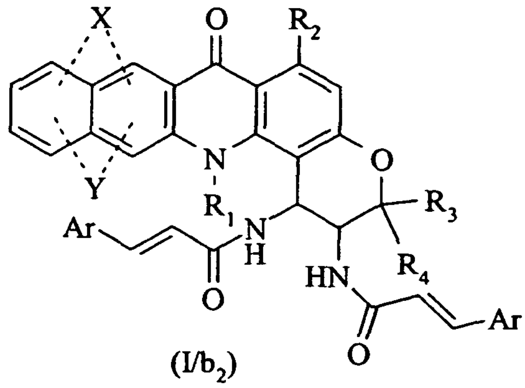

($I/b_2$)

dans lesquelles X, Y, $R_1$, $R_2$, $R_3$, $R_4$ et Ar sont tels que définis précédemment, composés de formule ($I/b_1$) ou ($I/b_2$) qui sont éventuellement soumis à l'action d'un composé de formule (XII) :

$R'_c$-Hal (XII)

dans laquelle Hal représente un halogène et $R'_c$ représente un groupement choisi parmi alkyle ($C_1$-$C_6$) linéaire ou ramifié, ou un groupement aryle, arylalkyle ($C_1$-$C_6$) linéaire ou ramifié, pour conduire aux composés de formule ($I/c_1$) ou ($I/c_2$) :

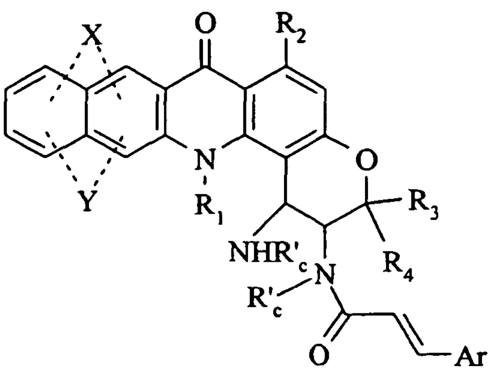

($I/c_1$)

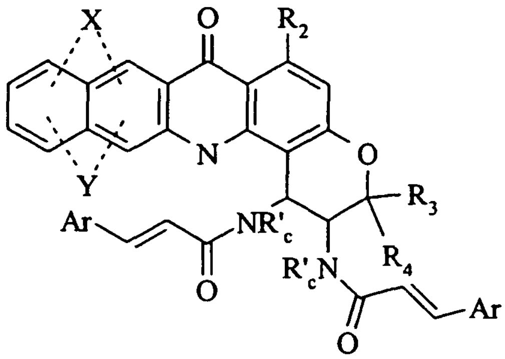

($I/c_2$)

dans lesquelles X, Y, $R_1$, $R_2$, $R_3$, $R_4$, $R'_c$ et Ar sont tels que définis précédemment,
l'ensemble des composés de formules (I/$a_1$), (I/$b_1$) et (I/$c_1$) et (I/$a_2$), (I/$b_2$) et (I/$c_2$) formant les composés de formules (I/$d_1$) et (I/$d_2$) respectivement,

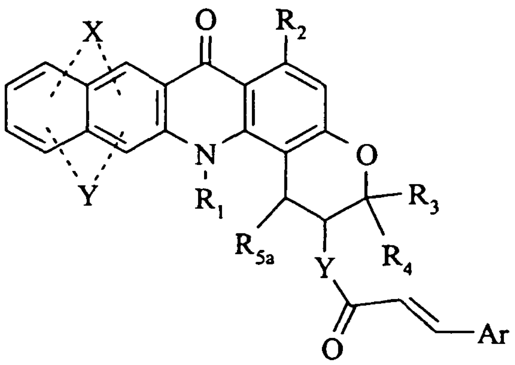

(I/$d_1$)

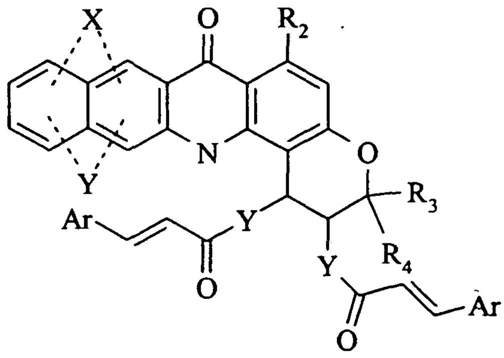

(I/$d_2$)

dans lesquelles X, Y, $R_1$, $R_2$, $R_3$, $R_4$, Y et Ar sont tels que définis dans la formule (I) et $R_{5a}$ représente un groupement hydroxy, $NH_2$ ou $NHR'_c$ dans lequel $R'_c$ est tel que défini précédemment,
les composés de formule (I/$d_1$) qui sont éventuellement soumis :

a) soit à l'action d'un agent alkylant, pour conduire aux composés de formule (I/e), cas particulier des composés de formule (I) :

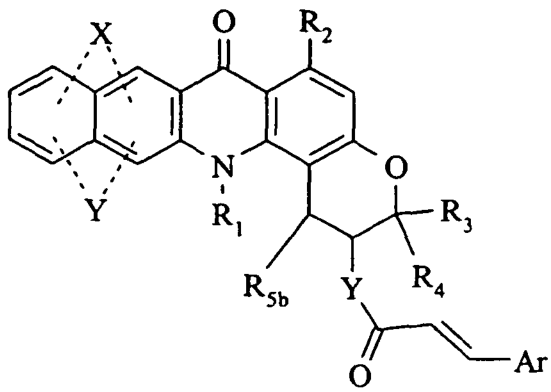

(I/e)

dans laquelle X, Y, $R_1$, $R_2$, $R_3$, $R_4$, Y et Ar sont tels que définis précédemment, et $R_{5b}$ représente un groupement $OR_c$, $NR_cR_d$ dans lesquels $R_c$ et $R_d$ sont tels que définis dans la formule (I),
b) soit à l'action d'un anhydride de formule (XIII) ou d'un chlorure d'acide de formule (XIV):

$(R_{10})_2O$ (XIII) $R_{10}$-Cl (XIV)

dans laquelle $R_{10}$ représente un groupement de formule $C(W_2)$-U-V ou $C(W_2)$-$W_3$-$T_1$ dans lesquelles $W_2$, $W_3$, U, V et $T_1$ sont tels que définis dans la formule (I), pour conduire aux composés de formule (I/f), cas particulier des composés de formule (I) :

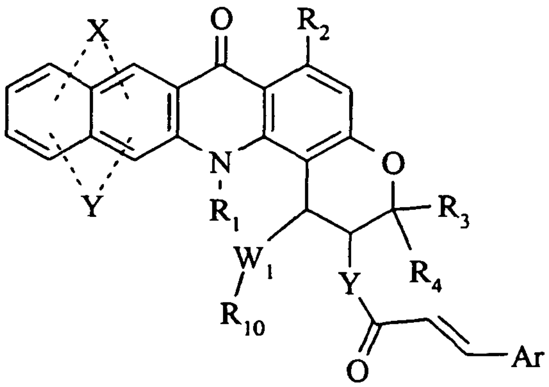

(I/f)

dans laquelle X, Y, $R_1$, $R_2$, $R_3$, $R_4$, Y, Ar et $R_{10}$ sont tels que définis précédemment et $W_1$ est tel que défini dans la formule (I),

les composés (I/a) à (I/f) formant l'ensemble des composés de l'invention qui sont purifiés, le cas échéant, selon une technique classique de purification, qui peuvent, si on le désire, être séparés en leurs différents isomères selon une technique classique de séparation et qui sont transformés, si on le souhaite, en leurs sels d'addition à un acide ou à une base pharmaceutiquement acceptable.

**11.** Compositions pharmaceutiques contenant comme principe actif au moins un composé selon l'une quelconque des revendications 1 à 9, en combinaison avec un ou plusieurs excipients ou véhicules inertes, non toxiques, pharmaceutiquement acceptable.

**12.** Compositions pharmaceutiques selon la revendication 11 contenant au moins un principe actif selon l'une quelconque des revendications 1 à 9 utiles en tant que médicaments, dans le traitement des cancers.

**Claims**

**1.** Compounds of formula (I):

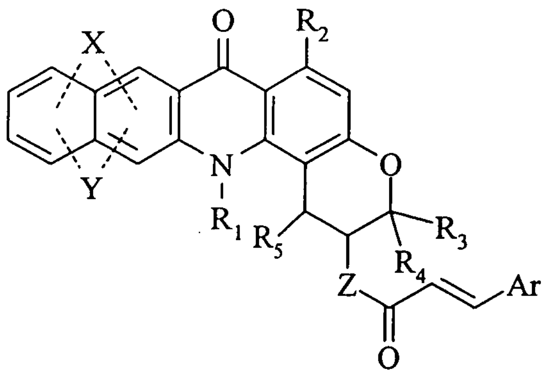

(I),

wherein :

• X and Y, which may be the same or different, represent, each independently of the other, a group selected from :

- hydrogen and halogen atoms,
- hydroxy, linear or branched ($C_1$-$C_6$)alkoxy, nitro, cyano, linear or branched ($C_1$-$C_6$)alkyl (optionally substituted by one or more groups selected from hydroxy and halogen) and linear or branched ($C_2$-$C_6$)alkenyl groups and
- a group of formula $-NR_aR_b$ wherein :

$R_a$ and $R_b$, which may be the same or different, represent, each independently of the other, a group selected from a hydrogen atom and a linear or branched ($C_1$-$C_6$)alkyl group,
or $R_a$ and $R_b$, together with the nitrogen atom carrying them, form a monocyclic, 5-to 7-membered heterocycle optionally containing within the cyclic system a second hetero atom selected from oxygen and nitrogen,
it being understood that the substituents X and Y may be present, independently of one another, on either of the two adjacent benzene rings,

- Z represents an oxygen atom or $NR_c$ wherein $R_c$ represents a group selected from a hydrogen atom, a linear or branched ($C_1$-$C_6$)alkyl group, an aryl group and an aryl-($C_1$-$C_6$)alkyl group in which the alkyl moiety is linear or branched,
- Ar represents an aryl or heteroaryl group,
- $R_1$ represents a hydrogen atom or a linear or branched ($C_1$-$C_6$)alkyl group,
- $R_2$ represents a group selected from a hydrogen atom, a linear or branched ($C_1$-$C_6$)-alkyl group, $-OR_a$ and $-NR_aR_b$ wherein $R_a$ and $R_b$ are as defined hereinbefore,
- $R_3$ and $R_4$, which may be the same or different, represent, independently of one other, a hydrogen atom or a linear or branched ($C_1$-$C_6$)alkyl group,
- $R_5$ represents a group selected from :

1) a hydrogen atom,
2) $OR_c$ and $NR_cR_d$ groups wherein :

$R_c$ is as defined hereinbefore and $R_d$ is as defined for $R_c$,

3) $W_1$-C($W_2$)-U-V wherein:

α) $W_1$ represents an oxygen atom or $NR_c$ (wherein $R_c$ is as defined hereinbefore),
β) $W_2$ represents an oxygen atom,
γ) U represents a single bond or a linear or branched ($C_1$-$C_8$)alkylene chain or a linear or branched ($C_2$-$C_8$)alkenylene chain,
δ) V represents a group selected from :

- a hydrogen atom,
- aryl and heteroaryl groups,
- $OR_c$, $CO_2R_c$, $COR_c$, $CONR'_aR'_b$, $NR'_aR'_b$, $N(R_c)$-$CO_2R'_c$ and $N(R_c)$-$COR'_c$ groups wherein $R_c$ is as defined hereinbefore, $R'_c$ is as defined for $R_e$, and $R'_a$ and $R'_b$, which may be the same or different, represent, each independently of the other, a group selected from a hydrogen atom, a linear or branched ($C_1$-$C_6$)alkyl group, an aryl group and an aryl-($C_1$-$C_6$)alkyl group in which the alkyl moiety is linear or branched or $R'_a$ and $R'_b$, together with the nitrogen atom carrying them, form a monocyclic, 5- to 7-membered heterocycle optionally containing within the cyclic system a second hetero atom selected from oxygen and nitrogen,

4) $W_1$-C($W_2$)-$W_3$-$T_1$ wherein :

α) $W_1$ and $W_2$ are as defined hereinbefore,
β) $W_3$ represents an oxygen atom or $NR_c$ wherein $R_c$ is as defined hereinbefore,
γ) $T_1$ represents a group selected from :

- a hydrogen atom,
- linear or branched ($C_1$-$C_6$)alkyl,
- linear or branched ($C_2$-$C_6$)alkenyl,
- aryl, aryl-($C_1$-$C_6$)alkyl in which the alkyl moiety is linear or branched,
- a linear or branched ($C_1$-$C_6$)alkylene chain and a linear or branched ($C_2$-$C_6$)alkenylene chain, each of which is substituted by a group $OR_c$ wherein $R_c$ is as defined hereinbefore or by $NR'_aR'_b$ wherein $R'_a$ and $R'_b$ are as defined hereinbefore,

5) Z-CO-CH=CHAr wherein Z and Ar are as defined hereinbefore,

their enantiomers and diastereoisomers, when they exist, and also addition salts thereof with a pharmaceutically acceptable acid or base, and also hydrates thereof and solvates thereof,
it being understood that :

aryl means a phenyl or naphthyl group optionally containing one or more, identical or different, substituents selected from linear or branched ($C_1$-$C_6$)alkyl (optionally substituted by one or more hydroxy or halogen groups), hydroxy, halogen, carboxy, nitro, amino, linear or branched mono($C_1$-$C_6$)alkylanaino, di($C_1$-$C_6$) alkylamino wherein each alkyl moiety may be linear or branched, linear or branched ($C_1$-$C_6$)alkoxy, linear or branched ($C_1$-$C_6$)acyl and linear or branched ($C_1$-$C_6$)alkyl-carbonyloxy,
heteroaryl means a 5- to 12-membered group which either is monocyclic and aromatic or is bicyclic with at least one of the rings being of aromatic character, and which contains one, two or three hetero atoms selected from oxygen, nitrogen and sulphur, it being understood that the heteroaryl group may be optionally substituted by one or more identical or different atoms or groups selected from halogen atoms and linear or branched ($C_1$-$C_6$)alkyl groups (optionally substituted by one or more hydroxy or halogen groups), hydroxy groups, linear or branched ($C_1$-$C_6$)alkoxy groups, and amino groups (optionally substituted by one or two linear or branched ($C_1$-$C_6$)alkyl groups).

**2.** Compounds of formula (I) according to claim 1, **characterised in that** X and Y represent a hydrogen atom, their enantiomers and diastereoisomers, and also addition salts thereof with a pharmaceutically acceptable acid or base.

**3.** Compounds of formula (I) according to either claim 1 or claim 2, **characterised in that** $R_1$, $R_3$ and $R_4$ represent a linear or branched ($C_1$-$C_6$)alkyl group, their enantiomers and diastereoisomers, and also addition salts thereof with a pharmaceutically acceptable acid or base.

**4.** Compounds of formula (I) according to any one of claims 1 to 3, **characterised in that** $R_2$ represents the group $-OR_a$ wherein $R_a$ is as defined for formula (I), their enantiomers and diastereoisomers, and also addition salts thereof with a pharmaceutically acceptable acid or base.

**5.** Compounds of formula (I) according to any one of claims 1 to 4, **characterised in that** $R_5$ represents the groups $-OR_c$ or $W_1$-C($W_2$)-U-V wherein $R_c$, $W_1$, $W_2$, U and V are as defined for formula (I), their enantiomers and diastereoisomers, and also addition salts thereof with a pharmaceutically acceptable acid or base.

**6.** Compounds of formula (I) according to any one of claims 1 to 5, **characterised in that** $R_5$ represents the group $-OR_c$ wherein $R_c$ represents a hydrogen atom or $R_5$ represents the group $W_1$-C($W_2$)-U-V wherein $W_1$ and $W_2$ each represent an oxygen atom, U represents a linear or branched ($C_1$-$C_8$)alkylene chain and V represents a hydrogen atom, their enantiomers and diastereoisomers, and also addition salts thereof with a pharmaceutically acceptable acid or base.

**7.** Compounds of formula (I) according to any one of claims 1 to 6, **characterised in that** Z represents an oxygen atom, their enantiomers and diastereoisomers, and also addition salts thereof with a pharmaceutically acceptable acid or base.

**8.** Compounds of formula (I) according to any one of claims 1 to 7, **characterised in that** Ar represents an optionally substituted phenyl group, their enantiomers and diastereoisomers, and also addition salts thereof with a pharmaceutically acceptable acid or base.

**9.** Compounds of formula (I) according to claim 1, which are :

➢ (±)-*cis*-2-cinnamoyloxy-1-hydroxy-6-methoxy-3,3,14-trimethyl-1,2,3,14-tetrahydro-1*H*-benzo[*b*]pyrano[3,2-*h*]acridin-7-one,
➢ (±)-*cis*-1-acetoxy-2-cinnamoyloxy-6-methoxy-3,3,14-trimethyl-1,2,3,14-tetrahydro-1*H*-benzo[*b*]pyrano[3,2-*h*]acridin-7-one,
➢ (±)-*cis*-2-(4-chlorocinnamoyloxy)-1-hydroxy-6-methoxy-3,3,14-trimethyl-1,2,3,14-tetrahydro-1*H*-benzo[*b*]pyrano[3,2-*h*]acridin-7-one,
➢ (±)-*cis*-1-acetoxy-2-(4-chlorocinnamoyloxy)-6-methoxy-3,3,14-trimethyl-1,2,3,14-tetrahydro-1*H*-benzo[*b*]pyrano[3,2-*h*]acridin-7-one,
➢ (±)-*cis*-2-(2-chlorocinnamoyloxy)-1-hydroxy-6-methoxy-3,3,14-trimethyl-1,2,3,14-tetrahydro-1*H*-benzo[*b*]pyrano[3,2-*h*]acridin-7-one,

➢ (±)-*cis*-1-acetoxy-2-(2-chlorocinnamoyloxy)-6-methoxy-3,3,14-trimethyl-1,2,3,14-tetrahydro-1*H*-benzo[*b*]pyrano[3,2-*h*]acridin-7-one,
➢ (±)-*cis*-2-(3-chlorocinnamoyloxy)-1-hydroxy-6-methoxy-3,3,14-trimethyl-1,2,3,14-tetrahydro-1*H*-benzo[*b*]pyrano[3,2-*h*]acridin-7-one,
➢ (±)-*cis*-2-(2,4-dichlorocinnamoyloxy)-1-hydroxy-6-methoxy-3,3,14-trimethyl-1,2,3,14-tetrahydro-1*H*-benzo[*b*]pyrano[3,2-*h*]acridin-7-one,
➢ (±)-*cis*-1-acetoxy-2-(2,4-dichlorocinnamoyloxy)-6-methoxy-3,3,14-trimethyl-1,2,3,14-tetrahydro-1*H*-benzo[*b*]pyrano[3,2-*h*]acridin-7-one,
➢ (±)-*cis*-2-(3,4-dichlorocinnamoyloxy)-1-hydroxy-6-methoxy-3,3,14-trimethyl-1,2,3,14-tetrahydro-1*H*-benzo[*b*]pyrano[3,2-*h*]acridin-7-one,
➢ (±)-*cis*-1-acetoxy-2-(3,4-dichlorocinnamoyloxy)-6-methoxy-3,3,14-trimethyl-1,2,3,14-tetrahydro-1*H*-benzo[*b*]pyrano[3,2-*h*]acridin-7-one,
➢ (±)-*cis*-2-(4-bromocinnamoyloxy)-1-hydroxy-6-methoxy-3,3,14-trimethyl-1,2,3,14-tetrahydro-1*H*-benzo[*b*]pyrano[3,2-*h*]acridin-7-one,
➢ (±)-*cis*-1-acetoxy-2-(4-bromocinnamoyloxy)-6-methoxy-3,3,14-trimethyl-1,2,3,14-tetrahydro-1*H*-benzo[*b*]pyrano[3,2-*h*]acridin-7-one,
➢ (±)-*cis*-1-hydroxy-6-methoxy-2-(4-methoxycinnamoyloxy)-3,3,14-trimethyl-1,2,3,14-tetrahydro-1*H*-benzo[*b*]pyrano[3,2-*h*]acridin-7-one,
➢ (±)-*cis*-1-hydroxy-6-methoxy-2-(4-nitrocinnamoyloxy)-3,3,14-trimethyl-1,2,3,14-tetrahydro-1*H*-benzo[*b*]pyrano[3,2-*h*]acridin-7-one,
➢ (±)-*cis*-1-acetoxy-6-methoxy-2-(4-nitrocinnamoyloxy)-3,3,14-trimethyl-1,2,3,14-tetrahydro-1*H*-benzo[*b*]pyrano[3,2-*h*]acridin-7-one,

their enantiomers and diastereoisomers, and also addition salts thereof with a pharmaceutically acceptable acid or base.

**10.** Process for the preparation of compounds of formula (I) according to claim 1, **characterised in that** there is used as starting material a compound of formula (II) :

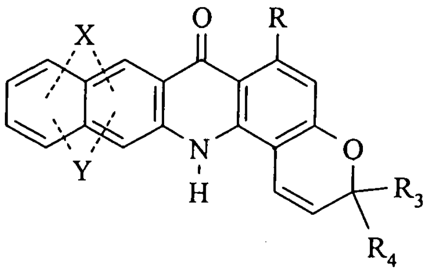

(II),

wherein X, Y, $R_3$ and $R_4$ are as defined hereinbefore and R represents a hydrogen atom, a hydroxy group or a linear or branched ($C_1$-$C_6$)alkyl group,
the nitrogen atom of which compound of formula (II) is substituted, or not, by the action of an alkyl halide or of a dialkyl sulphate in the presence of a deprotonating agent, in an aprotic polar solvent or under phase transfer conditions, allowing the compounds of formula (III) to be obtained:

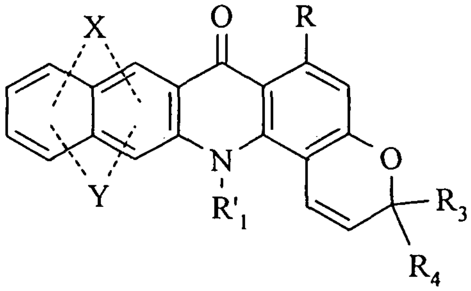

(III),

wherein X, Y, R, $R_3$ and $R_4$ are as defined hereinbefore and $R'_1$ represents a linear or branched $(C_1-C_6)$alkyl group, which compounds of formula (III) are subjected to the action of an alkylating agent under customary conditions of organic synthesis to yield the compounds of formula (IV) :

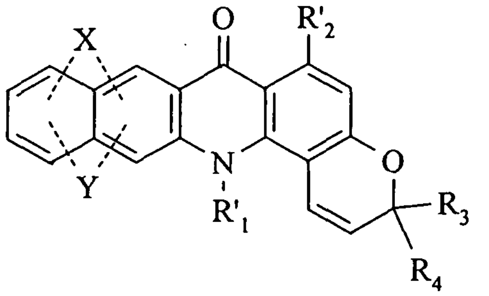

(IV),

wherein X, Y, $R'_1$, $R_3$ and $R_4$ are as defined hereinbefore and $R'_2$ represents a group selected from $OR'_a$ wherein $R'_a$ represents a linear or branched $(C_1-C_6)$alkyl group,
which compounds of formula (IV), in the case where $R'_2$ represents an alkoxy group, are treated with a compound of formula (V) :

$HNR_aR_b$ (V),

wherein $R_a$ and $R_b$ are as defined for formula (I),
to yield the compounds of formula (VI) :

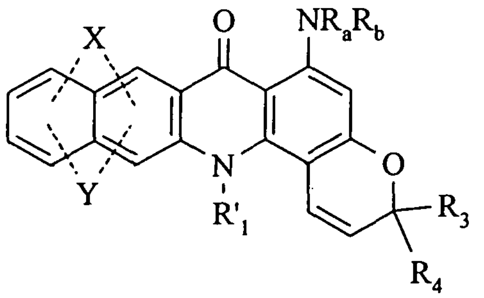

(VI),

wherein X, Y, $R'_1$, $R_3$, $R_4$, $R_a$ and $R_b$ are as defined hereinbefore,
the totality of the compounds of formulae (II), (III), (IV) and (VI) forming the compounds of formula (VII) :

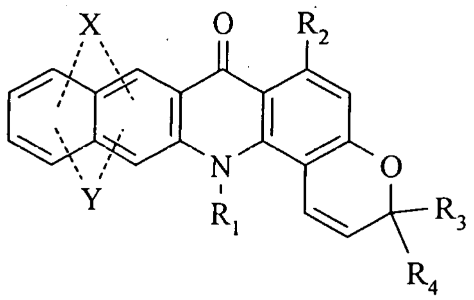

(VII),

wherein X, Y, $R_1$, $R_2$, $R_3$ and $R_4$ are as defined for formula (I),
which compounds of formula (VII) are subjected :

a) either to the action of osmium tetroxide in a polar medium and in the presence of 4-methylmorpholine N-oxide to yield the compounds of formula (VIII/a):

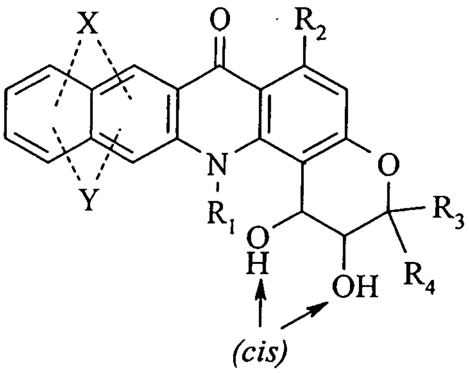

(VIII/a),

wherein X, Y, $R_1$, $R_2$, $R_3$ and $R_4$ are as defined hereinbefore,
b) or to the action of potassium permanganate in a polar medium and then to reductive conditions in the presence of $NaBH_4$ to yield the compounds of formula (VIII/b) :

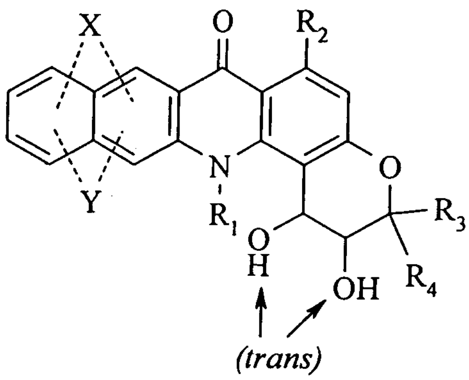

(VIII/b),

wherein X, Y, $R_1$, $R_2$, $R_3$ and $R_4$ are as defined hereinbefore,
the totality of the compounds of formulae (VIII/a) and (VIII/b) forming the compounds of formula (VIII) wherein the 2 alcohol groups may be of *cis* or *trans* configuration with respect to one another :

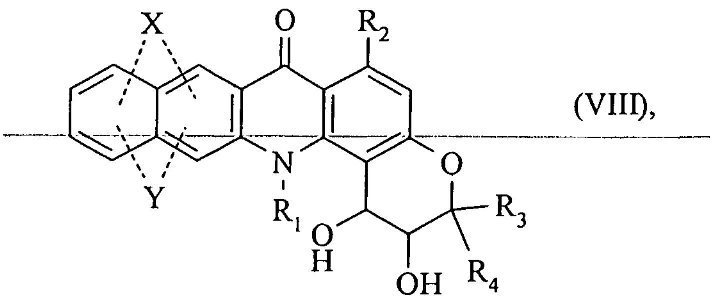

(VIII),

wherein X, Y, $R_1$, $R_2$, $R_3$ and $R_4$ are as defined hereinbefore,
which compounds of formula (VIII) are subjected to the action of one or 2 equivalents of an anhydride of formula (IX) or of an acid chloride of formula (X):

[Ar-CH=CH-C(O)]$_2$O (IX) Ar-HC=CH-C(O)-Cl (X),

wherein Ar is as defined for formula (I), to yield the compounds of formula (I/$a_1$) or (I/$a_2$), particular cases of the compounds of formula (I) :

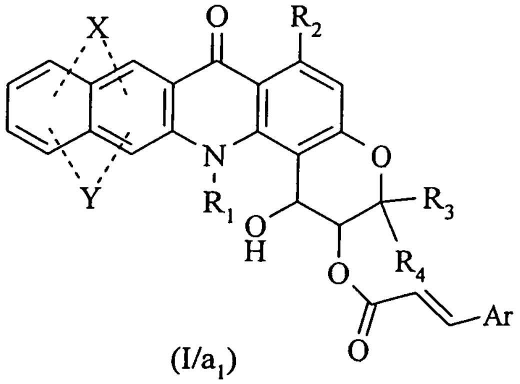
(I/$a_1$)

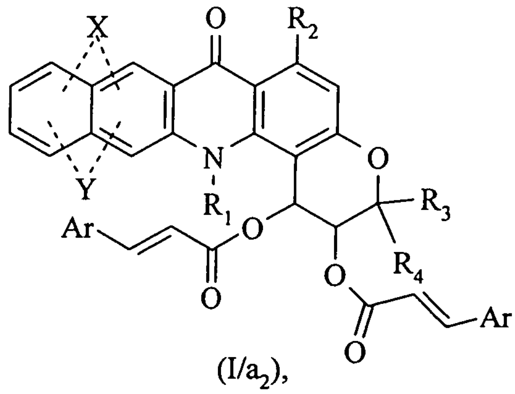
(I/$a_2$),

wherein X, Y, $R_1$, $R_2$, $R_3$, $R_4$ and Ar are as defined hereinbefore,
c) or to the action of $NaN_3$, in the presence of hydrogen peroxide, followed by a reduction step to yield the compounds of formula (XI) :

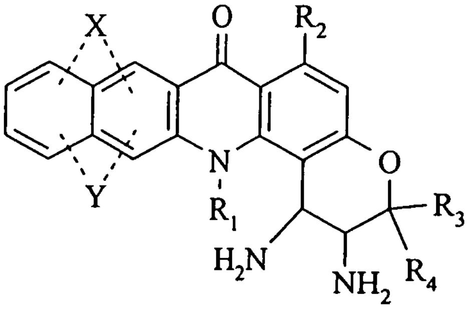
(XI),

wherein X, Y, $R_1$, $R_2$, $R_3$ and $R_4$ are as defined hereinbefore,
which compounds of formula (XI) are subjected to the action of compounds of formula (IX) or (X) as defined hereinbefore under the same conditions as for the compounds of formula (VIII) to yield the compounds of formula (I/$b_1$) or (I/$b_2$), particular cases of the compounds of formula (I) :

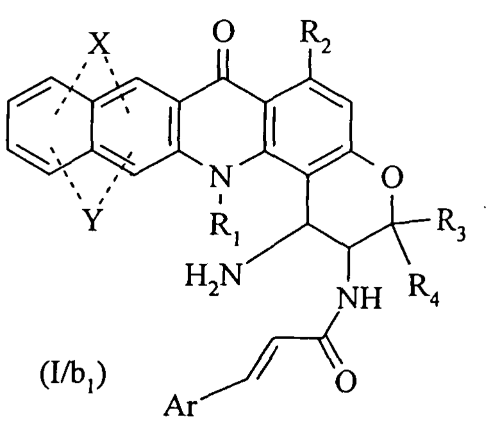
(I/$b_1$)

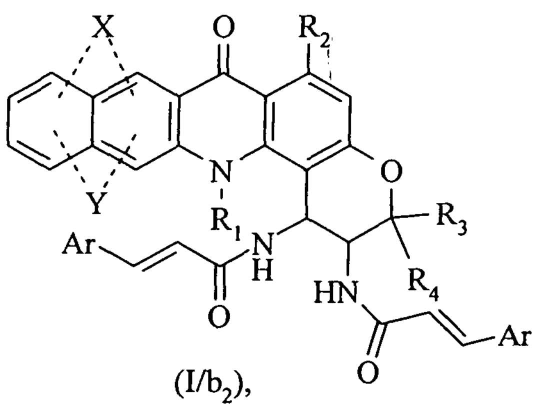
(I/$b_2$),

wherein X, Y, $R_1$, $R_2$, $R_3$, $R_4$ and Ar are as defined hereinbefore,
which compounds of formula (I/$b_1$) or (I/$b_2$) are optionally subjected to the action of a compound of formula (XII) :

$R'_c$-Hal (XII),

wherein Hal represents a halogen and R'$_c$ represents a group selected from linear or branched ($C_1$-$C_6$)alkyl, an aryl group and aryl-($C_1$-$C_6$)alkyl in which the alkyl moiety is linear or branched, to yield the compounds of formula (I/$c_1$) or (I/$c_2$) :

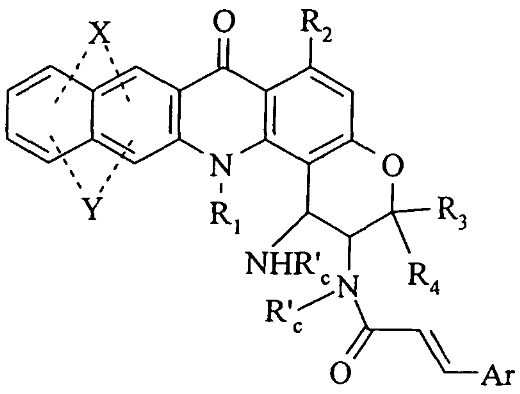

(I/$c_1$)

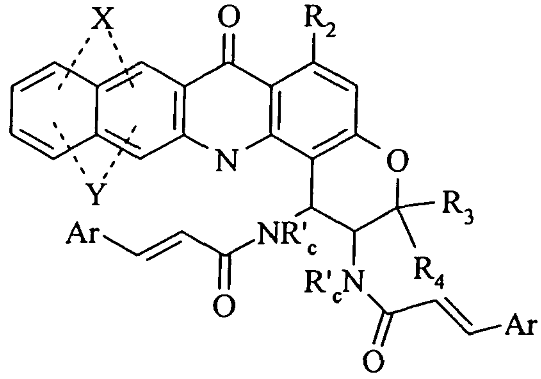

(I/$c_2$),

wherein X, Y, $R_1$, $R_2$, $R_3$, $R_4$, R'$_c$ and Ar are as defined hereinbefore,
the totality of the compounds of formulae (I/$a_1$), (I/$b_1$) and (I/$c_1$), and (I/$a_2$), (I/$b_2$) and (I/$c_2$) forming the compounds of formulae (I/$d_1$) and (I/$d_2$), respectively,

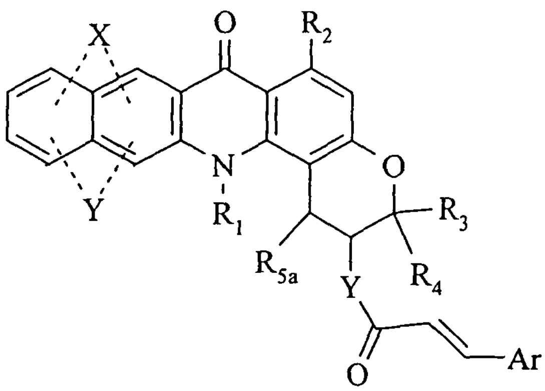

(I/$d_1$)

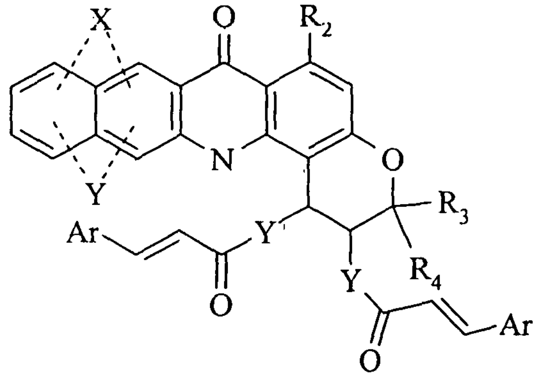

(I/$d_2$),

wherein X, Y, $R_1$, $R_2$, $R_3$, $R_4$, Y and Ar are as defined for formula (I) and $R_{5a}$ represents a hydroxy group, $NH_2$ or NHR'$_c$ wherein R'$_c$ is as defined hereinbefore,
which compounds of formula (I/$d_1$) are optionally subjected :

a) either to the action of an alkylating agent to yield the compounds of formula (I/e), a particular case of the compounds of formula (I) :

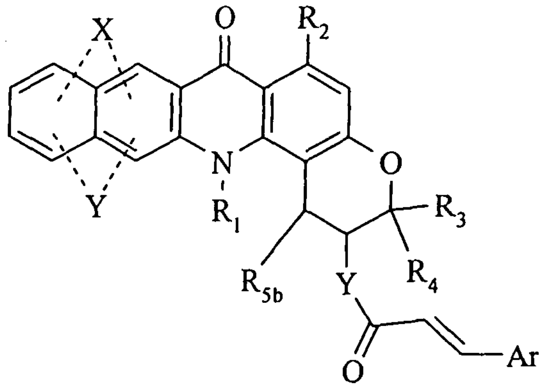

(I/e),

wherein X, Y, $R_1$, $R_2$, $R_3$, $R_4$, Y and Ar are as defined hereinbefore and $R_{5b}$ represents a group $OR_c$ or $NR_cR_d$ wherein $R_c$ and $R_d$ are as defined for formula (I),
b) or to the action of an anhydride of formula (XIII) or of an acid chloride of formula (XIV) :

$(R_{10})_2O$ (XIII) $R_{10}$ -Cl (XIV),

wherein $R_{10}$ represents a group of formula $C(W_2)$-U-V or $C(W_2)$-$W_3$-$T_1$ wherein $W_2$, $W_3$, U, V and $T_1$ are as defined for formula (I), to yield the compounds of formula (I/f), a particular case of the compounds of formula (I) :

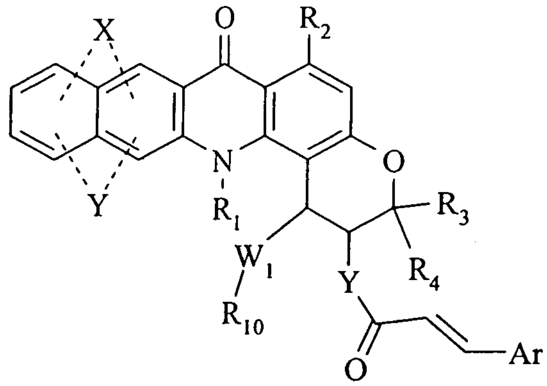

(I/f),

wherein X, Y, $R_1$, $R_2$, $R_3$, $R_4$, Y, Ar and $R_{10}$ are as defined hereinbefore and $W_1$ is as defined for formula (I),

the compounds (I/a) to (I/f) constituting the totality of the compounds of the invention, which are purified, if necessary, according to a conventional purification technique, which may be, if desired, separated into their different isomers according to a conventional separation technique and which are converted, if desired, into their addition salts with a pharmaceutically acceptable acid or base.

**11.** Pharmaceutical compositions comprising as active ingredient at least one compound according to any one of claims 1 to 9 in combination with one or more inert, non-toxic, pharmaceutically acceptable excipients or carriers.

**12.** Pharmaceutical compositions according to claim 11 comprising at least one active ingredient according to any one of claims 1 to 9 for use as medicaments in the treatment of cancers.

**Patentansprüche**

**1.** Verbindungen der Formel (I):

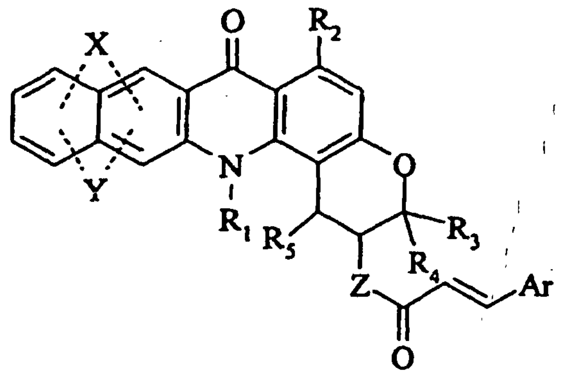

(I)

in der:

- X und Y, die gleichartig oder verschieden sind, unabhängig voneinander jeweils eine Gruppe bedeuten ausgewählt aus:

  - Wasserstoffatomen, Halogenatomen,
  - Hydroxygruppen, geradkettigen oder verzweigten ($C_1$-$C_6$)-Alkoxygruppen, Nitrogruppen, Cyanogruppen, geradkettigen oder verzweigten ($C_1$-$C_6$)-Alkylgruppen, die gegebenenfalls durch eine oder mehrere Gruppen ausgewählt aus Hydroxy und Halogen substituiert sind, und geradkettigen oder verzweigten ($C_2$-$C_6$)-Alkenylgruppen, oder
  - Gruppen der Formel $-NR_aR_b$, in der:

    $R_a$ und $R_b$, die gleichartig oder verschieden sind, unabhängig voneinander jeweils eine Gruppe bedeuten ausgewählt aus Wasserstoffatomen oder geradkettigen oder verzweigten ($C_1$-$C_6$)-Alkylgruppen,
    oder $R_a$ und $R_b$ gemeinsam mit dem sie tragenden Stickstoffatom einen monocyclischen Heterocyclus mit 5 bis 7 Kettengliedern bilden, der gegebenenfalls in dem cyclischen System ein zweites Heteroatom ausgewählt aus Sauerstoff und Stickstoff enthält,
    mit der Maßgabe, daß die Substituenten X und Y unabhängig voneinander an dem einen oder dem anderen der beiden benachbarten Benzolringe vorhanden sein können,

- Z ein Sauerstoffatom oder $NR_c$ bedeutet, worin $R_c$ eine Gruppe bedeutet ausgewählt aus dem Wasserstoffatom, geradkettigen oder verzweigten ($C_1$-$C_6$)-Alkylgruppen, Arylgruppen oder geradkettigen oder verzweigten Aryl-($C_1$-$C_6$)-alkylgruppen,
- Ar eine Aryl- oder Heteroarylgruppe bedeutet,
- $R_1$ ein Wasserstoffatom oder eine geradkettige oder verzweigte ($C_1$-$C_6$)-Alkylgruppe bedeutet,
- $R_2$ eine Gruppe bedeutet ausgewählt aus dem Wasserstoffatom, geradkettigen oder verzweigten ($C_1$-$C_6$)-Alkylgruppen oder Gruppen $-OR_a$ ; $-NR_aR_b$, worin $R_a$ und $R_b$ die oben angegebenen Bedeutungen besitzen,
- $R_3$ und $R_4$, die gleichartig oder verschieden sind, unabhängig voneinander ein Wasserstoffatom oder eine geradkettige oder verzweigte ($C_1$-$C_6$)-Alkylgruppe bedeuten,
- $R_5$ eine Gruppe bedeutet ausgewählt aus:

  1) dem Wasserstoffatom,
  2) Gruppen $OR_c$ und $NR_cR_d$, in denen:

     - $R_c$ die oben angegebenen Bedeutungen besitzt und $R_d$ die gleichen Bedeutungen aufweist wie $R_c$,

  3) $W_1$-C($W_2$)-U-V, in der:

     α) $W_1$ ein Sauerstoffatom oder $NR_c$ darstellt (worin $R_c$ die oben angegebenen Bedeutungen besitzt),
     β) $W_2$ ein Sauerstoffatom darstellt,
     γ) U eine Einfachbindung oder eine geradkettige oder verzweigte ($C_1$-$C_8$)-Alkylenkette oder eine geradkettige oder verzweigte ($C_2$-$C_8$)-Alkenylenkette darstellt,
     δ) V eine Gruppe darstellt ausgewählt aus:

- dem Wasserstoffatom,
- Aryl- und Heteroarylgruppen,
- Gruppen $OR_c$, $CO_2R_c$, $COR_c$, $CONR'_aR'_b$, $NR'_aR'_b$, $N(R_c)-CO_2R'_c$, N(Rc)-COR'$_c$, in denen $R_c$ die oben angegebenen Bedeutungen besitzt, $R'_c$ die gleichen Bedeutungen besitzt wie $R_c$ und $R'_a$ und $R'_b$, die gleichartig oder verschieden sind, unabhängig voneinander jeweils eine Gruppe bedeuten ausgewählt aus dem Wasserstoffatom, geradkettigen oder verzweigten ($C_1$-$C_6$)-Alkylgruppen, Arylgruppen, geradkettigen oder verzweigten Aryl-($C_1$-$C_6$)-alkylgruppen, oder $R'_a$ und $R'_b$ gemeinsam mit dem sie tragenden Stickstoffatom einen monocyclischen Heterocyclus mit 5 bis 7 Kettengliedern bilden, der gegebenenfalls in dem cyclischen System ein zweites Heteroatom ausgewählt aus Sauerstoff und Stickstoff enthält,

4) $W_1$-C($W_2$)-$W_3$-$T_1$, in der:

α) $W_1$ und $W_2$ die oben angegebenen Bedeutungen besitzen,
β) $W_3$ ein Sauerstoffatom oder $NR_c$ darstellt, worin $R_c$ die oben angegebenen Bedeutungen besitzt,
γ) $T_1$ eine Gruppe darstellt ausgewählt aus:

- dem Wasserstoffatom,
- geradkettigem oder verzweigtem ($C_1$-$C_6$)-Alkyl,
- geradkettigem oder verzweigtem ($C_2$-$C_6$)-Alkenyl,
- Aryl oder geradkettigem oder verzweigtem Aryl-($C_1$-$C_6$)-alkyl,
- geradkettigen oder verzweigten ($C_1$-$C_6$)-Alkylenketten oder geradkettigen oder verzweigten ($C_2$-$C_6$)-Alkenylenketten, die jeweils substituiert sind durch eine Gruppe $OR_c$, worin $R_c$ die oben angegebenen Bedeutungen besitzt oder durch eine Gruppe $NR'_aR'_b$, worin $R'_a$ und $R'_b$ die oben angegebenen Bedeutungen besitzen, und

5) Z-CO-CH=CHAr, worin Z und Ar die oben angegebenen Bedeutungen besitzen,

deren Enantiomere, Diastereoisomere, falls diese existieren, sowie deren Additionssalze mit einer pharmazeutisch annehmbaren Säure oder Base sowie ihre Hydrate und Solvate,
mit der Maßgabe, daß man:

unter Aryl eine Phenyl- oder Naphthylgruppe versteht, die gegebenenfalls einen oder mehrere gleichartige oder verschiedenartige Substituenten aufweist, ausgewählt aus geradkettigem oder verzweigtem ($C_1$-$C_6$)-Alkyl, welches gegebenenfalls durch eine oder mehrere Hydroxygruppen oder Halogenatome substituiert ist, Hydroxy, Halogen, Carboxy, Nitro, Amino, geradkettigem oder verzweigtem ($C_1$-$C_6$)-Monoalkylamino oder geradkettigem oder verzweigtem Di-($C_1$-$C_6$)-alkylamino, geradkettigem oder verzweigtem ($C_1$-$C_6$)-Alkoxy, geradkettigem oder verzweigtem ($C_1$-$C_6$)-Acyl und geradkettigem oder verzweigtem ($C_1$-$C_6$)-Alkylcarbonyloxy,
unter Heteroaryl eine Gruppe mit 5 bis 12 Kettengliedern versteht, die entweder monocyclisch aromatisch oder bicyclisch, wobei mindestens einer der Ringe einen aromatischen Charakter besitzt, ist und ein, zwei oder drei Heteroatome enthält ausgewählt aus Sauerstoff, Stickstoff oder Schwefel, wobei es sich versteht, daß die Heteroarylgruppe gegebenenfalls durch ein oder mehrere gleichartige oder verschiedenartige Atome oder Gruppen substituiert sein kann, ausgewählt aus Halogenatomen und geradkettigen oder verzweigten ($C_1$-$C_6$)-Alkylgruppen, die gegebenenfalls durch eine oder mehrere Hydroxygruppen oder Halogenatome substituiert sind, Hydroxygruppen, geradkettigen oder verzweigten ($C_1$-$C_6$)-Alkoxygruppen oder Aminogruppen (gegebenenfalls substituiert durch eine oder zwei geradkettige oder verzweigte ($C_1$-$C_6$)-Alkylgruppen).

2. Verbindungen der Formel (I) nach Anspruch 1, **dadurch gekennzeichnet, daß** X und Y ein Wasserstoffatom bedeuten, deren Enantiomere, Diastereoisomere sowie deren Additionssalze mit einer pharmazeutisch annehmbaren Säure oder Base.

3. Verbindungen der Formel (I) nach einem der Ansprüche 1 bis 2, **dadurch gekennzeichnet, daß** $R_1$, $R_3$ und $R_4$ eine geradkettige oder verzweigte ($C_1$-$C_6$)-Alkylgruppe bedeuten, deren Enantiomere, Diastereoisomere sowie deren Additionssalze mit einer pharmazeutisch annehmbaren Säure oder Base.

4. Verbindungen der Formel (I) nach einem der Ansprüche 1 bis 3, **dadurch gekennzeichnet, daß** $R_2$ die Gruppe

$-OR_a$ bedeutet, in der $R_a$ die bezüglich der Formel (I) angegebenen Bedeutungen besitzt, deren Enantiomere, Diastereoisomere sowie deren Additionssalze mit einer pharmazeutisch annehmbaren Säure oder Base.

**5.** Verbindungen der Formel (I) nach enem der Ansprüche 1 bis 4, **dadurch gekennzeichnet, daß** $R_5$ eine Gruppe $-OR_c$ oder $W_1$-C($W_2$)-U-V darstellt, worin $R_c$, $W_1$, $W_2$, U und V die bezüglich der Formel (I) angegebenen Bedeutungen besitzen, deren Enantiomere, Diastereoisomere sowie deren Additionssalze mit einer pharmazeutisch annehmbaren Säure oder Base.

**6.** Verbindungen der Formel (I) nach einem der Ansprüche 1 bis 5, **dadurch gekennzeichnet, daß** $R_5$ die Gruppe $-OR_c$ darstellt, worin $R_c$ ein Wasserstoffatom bedeutet oder $R_5$ die Gruppe $W_1$-C($W_2$)-U-V darstellt, in der $W_1$ und $W_2$ jeweils ein Sauerstoffatom darstellen, U eine geradkettige oder verzweigte ($C_1$-$C_8$)-Alkylenkette bedeutet und V ein Wasserstoffatom darstellt, deren Enantiomere, Diastereoisomere sowie deren Additionssalze mit einer pharmazeutisch annehmbaren Säure oder Base.

**7.** Verbindungen der Formel (I) nach einem der Ansprüche 1 bis 6, **dadurch gekennzeichnet, daß** Z ein Sauerstoffatom darstellt, deren Enantiomere, Diastereoisomere sowie deren Additionssalze mit einer pharmazeutisch annehmbaren Säure oder Base.

**8.** Verbindungen der Formel (I) nach einem der Ansprüche 1 bis 7, **dadurch gekennzeichnet, daß** Ar eine gegebenenfalls substituierte Phenylgruppe darstellt, deren Enantiomere, Diastereoisomere sowie deren Additionssalze mit einer pharmazeutisch annehmbaren Säure oder Base.

**9.** Verbindungen der Formel (I) nach Anspruch 1, nämlich:

➢ (±)-*cis*-2-Cinnamoyloxy-1-hydroxy-6-methoxy-3,3,14-trimethyl-1,2,3,14-tetrahydro-1*H*-benzo[*b*]pyrano [3,2-*h*]acridin-7-on,
➢ (±)-*cis*-1-Acetoxy-2-cinnamoyloxy-6-methoxy-3,3,14-trimethyl-1,2,3,14-tetrahydro-1*H*-benzo[*b*]pyrano [3,2-*h*]acridin-7-on,
➢ (±)-*cis*-2-(4-Chlorcinnamoyloxy)-1-hydroxy-6-methoxy-3,3,14-trimethyl-1,2,3,14-tetrahydro-1*H*-benzo[*b*] pyrano[3.2-*h*]acridin-7-on,
➢ (±)-*cis*-1-Acetoxy-2-(4-chlorcinnamoyloxy)-6-methoxy-3,3,14-trimethyl-1,2,3,14-tetrahydro-1*H*-benzo[*b*]pyrano[3,2-*h*]acridin-7-on,
➢ (±)-*cis*-2-(2-Chlorcinnamoyloxy)-1-hydroxy-6-methoxy-3,3,14-trimethyl-1,2,3,14-tetrahydro-1*H*-benzo[*b*] pyrano[3,2-*h*]acridin-7-on,
➢ (±)-*cis*-1-Acetoxy-2-(2-chlorcinnamoyloxy)-6-methoxy-3,3,14-trimethyl-1,2,3,14-tetrahydro-1*H*-benzo[*b*]pyrano[3,2-*h*]acridin-7-on,
➢ (±)-*cis*-2-(3-Chlorcinnamoyloxy)-1-hydroxy-6-methoxy-3,3,14-trimethyl-1,2,3,14-tetrahydro-1*H*-benzo[*b*] pyrano[3,2-*h*]acridin-7-on,
➢ (±)-*cis*-2-(2,4-Dichlorcinnamoyloxy)-1-hydroxy-6-methoxy-3,3,14-trimethyl-1,2,3,14-tetrahydro-1*H*-benzo [*b*]pyrano[3,2-*h*]acridin-7-on,
➢ (±)-*cis*-1-Acetoxy-2-(2,4-dichlorcinnamoyloxy)-6-methoxy-3,3,14-trimethyl-1,2,3,14-tetrahydro-1*H*-benzo [*b*]pyrano[3,2-*h*]acridin-7-on,
➢ (±)-*cis*-2-(3,4-Dichlorcinnamoyloxy)-1-hydroxy-6-methoxy-3,3,14-trimethyl-1,2,3,14-tetrahydro-1*H*-benzo [*b*]pyrano[3,2-*h*]acridin-7-on,
➢ (±)-*cis*-1-Acetoxy-2-(3,4-dichlorcinnamoyloxy)-6-methoxy-3,3,14-trimethyl-1,2,3,14-tetrahydro-1*H*-benzo [*b*]pyrano[3,2-*h*]acridin-7-on,
➢ (±)-*cis*-2-(4-Bromcinnamoyloxy)-1-hydroxy-6-methoxy-3,3,14-trimethyl-1,2,3,14-tetrahydro-1*H*-benzo[*b*] pyrano[3,2-*h*]acridin-7-on,
➢ (±)-*cis*-1-Acetoxy-2-(4-bromcinnamoyloxy)-6-methoxy-3,3,14-trimethyl-1,2,3,14-tetrahydro-1*H*-benzo[*b*] pyrano[3,2-*h*]acridin-7-on,
➢ (±)-*cis*-1-Hydroxy-6-methoxy-2-(4-methoxycinnamoyloxy)-3,3,14-trimethyl-1,2,3,14-tetrahydro-1*H*-benzo [*b*]pyrano[3,2-*h*]acridin-7-on,
➢ (±)-*cis*-1-Hydroxy-6-methoxy-2-(4-nitrocinnamoyloxy)-3,3,14-trimethyl-1,2,3,14-tetrahydro-1*H*-benzo[*b*]pyrano[3,2-*h*]acridin-7-on,
➢ (±)-*cis*-1-Acetoxy-6-methoxy-2-(4-nitrocinnamoyloxy)-3,3,14-trimethyl-1,2,3,14-tetrahydro-1*H*-benzo[*b*]pyrano[3,2-*h*]acridin-7-on,

deren Enantiomere, Diastereoisomere sowie deren Additionssalze mit einer pharmazeutisch annehmbaren Säure

oder Base.

**10.** Verfahren zur Herstellung der Verbindungen der Formel (I) nach Anspruch 1, **dadurch gekennzeichnet, daß** man als Ausgangsprodukt eine Verbindung der Formel (II) verwendet:

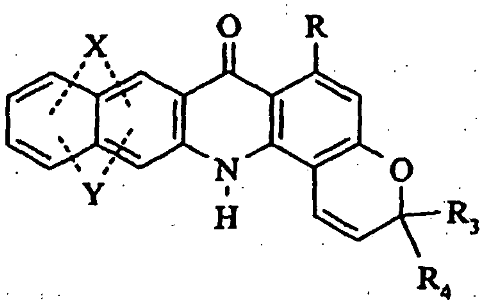

(II)

in der X, Y, $R_3$ und $R_4$ die oben angegebenen Bedeutungen besitzen und R ein Wasserstoffatom, eine Hydroxygruppe oder eine geradkettige oder verzweigte ($C_1$-$C_6$)-Alkylgruppe darstellt,
von welcher Verbindung der Formel (II) das Stickstoffatom gegebenenfalls durch Einwirkung eines Alkylhalogenids oder eines Dialkylsulfats in Gegenwart eines Deprotonierungsmittels, in einem aprotischen polaren Lösungsmittel oder unter Phasentransferbedingungen substituiert wird zur Bildung der Verbindungen der Formel (III):

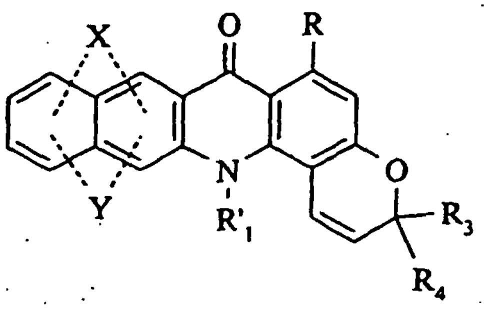

(III)

in der X, Y, R, $R_3$ und $R_4$ die oben angegebenen Bedeutungen besitzen und $R'_1$ eine geradkettige oder verzweigte ($C_1$-$C_6$)-Alkylgruppe darstellt,
welche Verbindungen der Formel (III) der Einwirkung eines Alkylierungsmittels unter klassischen Bedingungen der organischen Synthese unterworfen werden zur Bildung der Verbindungen der Formel (IV):

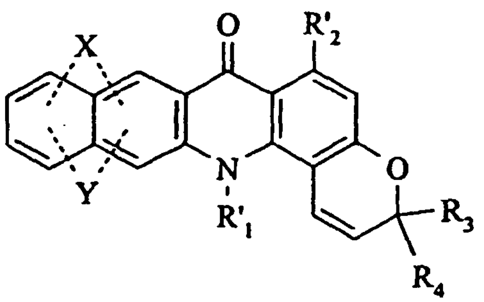

(IV)

in der X, Y, $R'_1$, $R_3$ und $R_4$ die oben angegebenen Bedeutungen besitzen und $R'_2$ eine Gruppe bedeutet ausgewählt aus $OR'_a$, worin $R'_a$ eine geradkettige oder verzweigte ($C_1$-$C_6$)-Alkylgruppe darstellt,
welche Verbindungen der Formel (IV) in dem Fall, da $R'_2$ eine Alkoxygruppe bedeutet, mit einer Verbindung der Formel (V) behandelt werden:

$HNR_aR_b$ (V)

in der $R_a$ und $R_b$ die bezüglich der Formel (I) angegebenen Bedeutungen besitzen, zur Bildung der Verbindungen der Formel (VI):

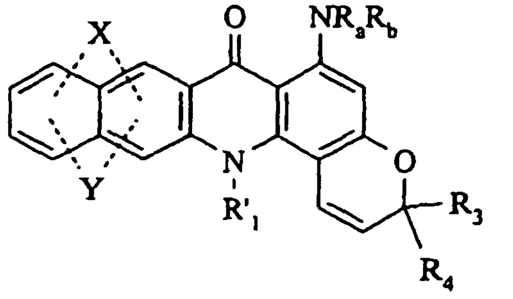

(VI)

in der X, Y, $R'_1$, $R_3$, $R_4$, $R_a$ und $R_b$ die oben angegebenen Bedeutungen besitzen,
wobei die Gesamtheit der Verbindungen der Formeln (II), (III), (IV) und (VI) die Verbindungen der Formel (VII) bilden:

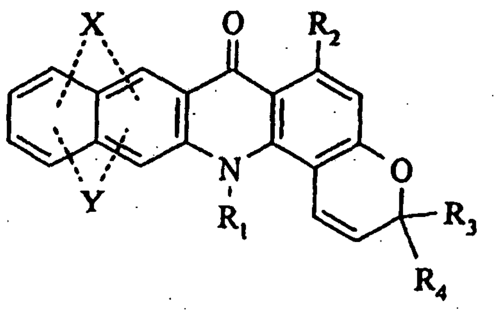

(VII)

in der X, Y, $R_1$, $R_2$, $R_3$ und $R_4$ die bezüglich der Formel (I) angegebenen Bedeutungen besitzen,
welche Verbindungen der Formel (VII):

a) entweder der Einwirkung von Osmiumtetroxid in polarem Medium und in Gegenwart von 4-Methylmorpholin-N-oxid unterworfen werden zur Bildung der Verbindungen der Formel (VIII/a):

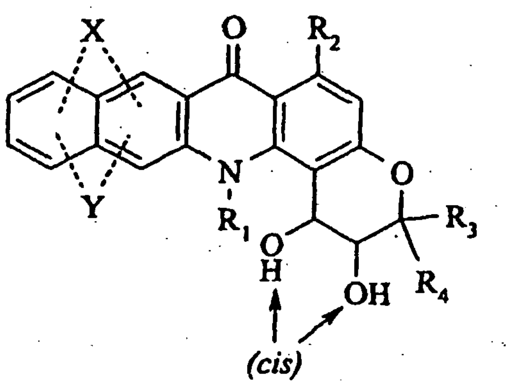

(VIII/a)

in der X, Y, $R_1$, $R_2$, $R_3$ und $R_4$ die oben angegebenen Bedeutungen besitzen,
b) oder der Einwirkung von Kaliumpermanganat in polarem Medium und dann reduzierenden Bedingungen in Gegenwart von $NaBH_4$ unterworfen werden zur Bildung der Verbindungen der Formel (VIII/b):

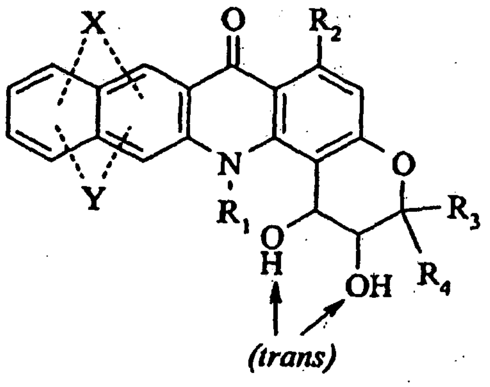


(VIII/b)

in der X, Y, $R_1$, $R_2$, $R_3$ und $R_4$ die oben angegebenen Bedeutungen besitzen,
wobei die Gesamtheit der Verbindungen der Formel (VIII/a) und (VIII/b) die Verbindungen der Formel (VIII) bilden, deren 2 Alkoholgruppen in bezug aufeinander in der *cis*- oder *trans*-Konfiguration vorliegen können:

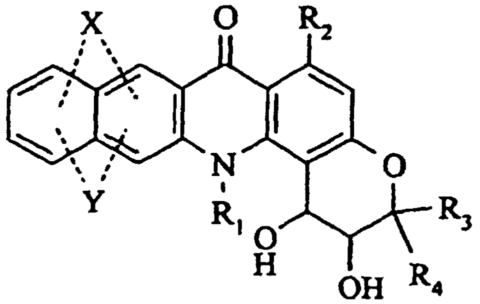

(VIII)

in der X, Y, $R_1$, $R_2$, $R_3$ und $R_4$ die oben angegebenen Bedeutungen besitzen,
welche Verbindungen der Formel (VIII) der Einwirkung von einem oder 2 Äquivalenten eines Anhydrids der Formel (IX) oder eines Säurechlorids der Formel (X) unterworfen werden:

$[Ar\text{-}CH{=}CH\text{-}C(O)]_2O$ (IX) $Ar\text{-}HC{=}CH\text{-}C(O)\text{-}Cl$ (X)

in denen Ar die bezüglich der Formel (I) angegebenen Bedeutungen besitzt, zur Bildung der Verbindungen der Formeln ($I/a_1$) oder ($I/a_2$), Sonderfällen der Verbindungen der Formel (I):

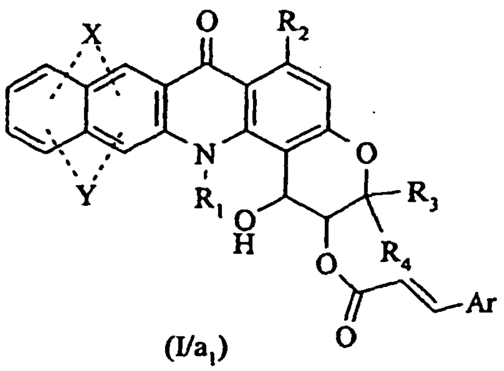

($I/a_1$)

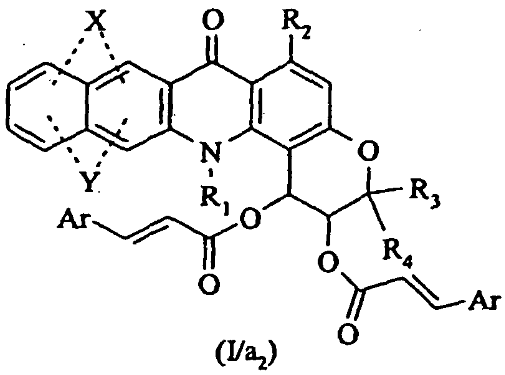

($I/a_2$)

in denen X, Y, $R_1$, $R_2$, $R_3$, $R_4$ und Ar die oben angegebenen Bedeutungen besitzen,
c) oder der Einwirkung von $NaN_3$ in Gegenwart von Wasserstoffperoxid unterworfen werden gefolgt von einer Stufe der Reduktion zur Bildung der Verbindungen der Formel (XI):

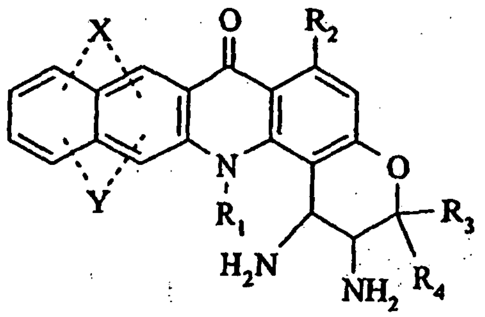

(XI)

in der X, Y, $R_1$, $R_2$, $R_3$ und $R_4$ die oben angegebenen Bedeutungen besitzen, welche Verbindungen der Formel (XI) der Einwirkung der Verbindungen der Formel (IX) oder (X), wie sie oben definiert worden sind, bei den gleichen Bedingungen wie die Verbindungen der Formel (VIII) unterworfen werden zur Bildung der Verbindungen der Formeln ($I/b_1$) oder ($I/b_2$), Sonderfällen der Verbindungen der Formel (I):

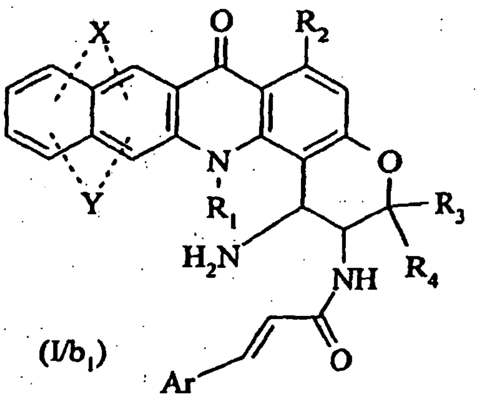

($I/b_1$)

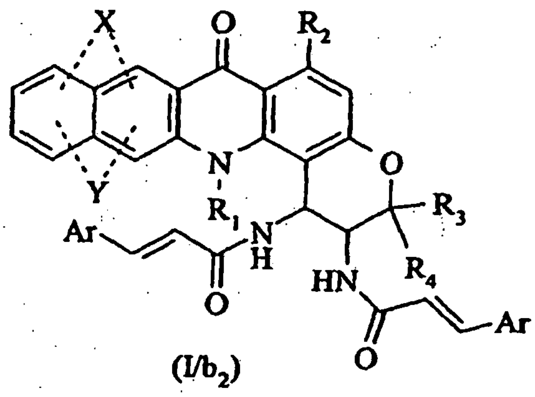

($I/b_2$)

in denen X, Y, $R_1$, $R_2$, $R_3$, $R_4$ und Ar die oben angegebenen Bedeutungen besitzen,
welche Verbindungen der Formeln ($I/b_1$) oder ($I/b_2$) gegebenenfalls der Einwirkung einer Verbindung der Formel (XII) unterworfen werden:

$$R'_c - Hal \qquad (XII)$$

in der Hal ein Halogenatom darstellt und $R'_c$ eine Gruppe bedeutet ausgewählt aus geradkettigem oder verzweigtem ($C_1$-$C_6$)-Alkyl oder einer Arylgruppe oder einer geradkettigen oder verzweigten Aryl-($C_1$-$C_6$)-alkylgruppe, zur Bildung der Verbindungen der Formel ($I/c_1$) oder ($I/c_2$):

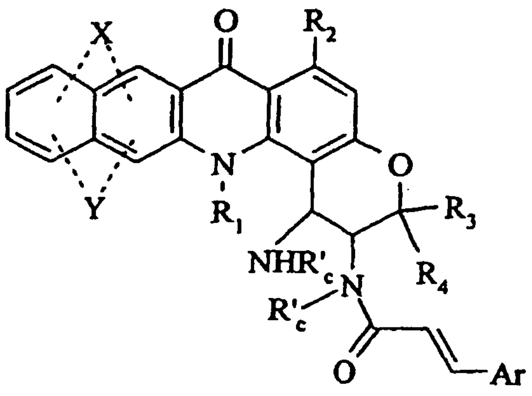

(I/c$_1$)

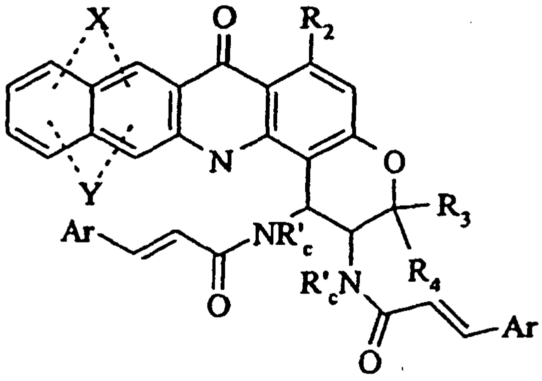

(I/c$_2$)

in denen X, Y, $R_1$, $R_2$, $R_3$, $R_4$, $R'_c$ und Ar die oben angegebenen Bedeutungen besitzen, wobei die Gesamtheit der Verbindungen der Formeln (I/$a_1$). (I/$b_1$) und (I/$c_1$) und (I/$a_2$), (I/$b_2$) und (I/$c_2$) die Verbindungen der Formeln (I/$d_1$ bzw. (I/$d_2$) bilden:

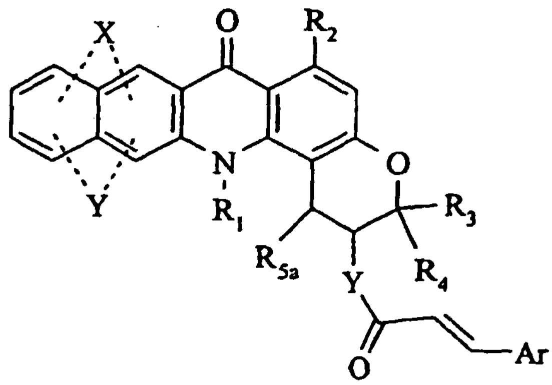

(I/d$_1$)

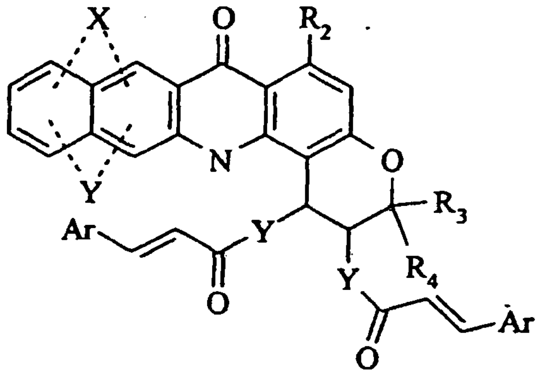

(I/d$_2$)

in denen X, Y, $R_1$, $R_2$, $R_3$, $R_4$, Y und Ar die bezüglich der Formel (I) angegebenen Bedeutungen besitzen und $R_{5a}$ eine Hydroxygruppe, $NH_2$ oder $NHR'_c$ darstellt, worin $R'_c$ die oben angegebenen Bedeutungen besitzt, welche Verbindungen der Formel (I/$d_1$) gegebenenfalls:

a) entweder der Einwirkung eines Alkylierungsmittels unterworfen werden zur Bildung der Verbindungen der Formel (I/e), einem Sonderfall der Verbindungen der Formel (I):

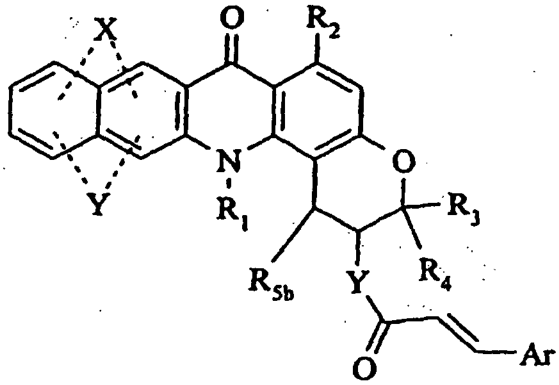

(I/e)

in der X, Y, $R_1$, $R_2$, $R_3$, $R_4$, Y und Ar die oben angegebenen Bedeutungen besitzen und $R_{5b}$ eine Gruppe $OR_c$ oder $NR_cR_d$ darstellt, worin $R_c$ und $R_d$ die bezüglich der Formel (I) angegebenen Bedeutungen besitzen,
b) oder der Einwirkung eines Anhydrids der Formel (XIII) oder eines Säurechlorids der Formel (XIV) unterworfen werden:

$(R_{10})_2O$ (XIII) $R_{10}$-Cl (XIV)

in denen $R_{10}$ eine Gruppe der Formel $C(W_2)$-U-V oder $C(W_2)$-$W_3$-$T_1$ darstellt, worin $W_2$, $W_3$, U, V und T, die bezüglich der Formel (I) angegebenen Bedeutungen besitzen, zur Bildung der Verbindungen der Formel (I/f), einem Sonderfall der Verbindungen der Formel (I):

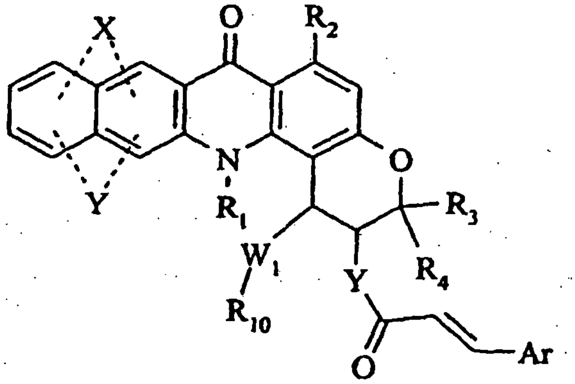

(I/f)

in der X, Y, $R_1$, $R_2$, $R_3$, $R_4$, Y, Ar und $R_{10}$ die oben angegebenen Bedeutungen besitzen und W, die bezüglich der Formel (I) angegebenen Bedeutungen besitzt,

welche Verbindungen der Formeln (I/a) bis (I/f), welche die Gesamtheit der erfindungsgemäßen Verbindungen bilden, gegebenenfalls mit Hilfe einer klassischen Reinigungsmethode gereinigt werden können, gewünschtenfalls mit Hilfe einer klassischen Trennmethode in ihre verschiedenen Isomeren aufgetrennt werden können und gewünschtenfalls in ihre Additionssalze mit einer pharmazeutisch annehmbaren Säure oder Base überführt werden können.

**11.** Pharmazeutische Zubereitungen, enthaltend als Wirkstoff mindestens eine Verbindung nach einem der Ansprüche 1 bis 9 in Kombination mit einem oder mehreren inerten, nichttoxischen, pharmazeutisch annehmbaren Trägermaterialien oder Hilfsstoffen.

**12.** Pharmazeutische Zubereitungen nach Anspruch 11, enthaltend mindestens einen Wirkstoff nach einem der Ansprüche 1 bis 9 nützlich als Arzneimittel für die Behandlung von Krebs.

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description


- EP 1042326 A
- EP 1061081 A
- EP 1297835 A


### Littérature non-brevet citée dans la description


- *J. Pharm.. Sci,* 1966, vol. 55 (8), 758-768
- *J. Med. Chem.,* 1996, vol. 39, 4762-4766
- *Cancer Res.,* 1987, vol. 47, 939-942